(19) Europäisches Patentamt
European Patent Office
Office européen des brevets

(11) **EP 4 023 147 B1**

(12) **FASCICULE DE BREVET EUROPEEN**

(45) Date de publication et mention
de la délivrance du brevet:
**15.11.2023 Bulletin 2023/46**

(21) Numéro de dépôt: **21217909.7**

(22) Date de dépôt: **28.12.2021**

(51) Classification Internationale des Brevets (IPC):
**A61B 5/00** *(2006.01)* **A61B 5/02** *(2006.01)*
**A61B 5/026** *(2006.01)*

(52) Classification Coopérative des Brevets (CPC):
**A61B 5/0059; A61B 5/02007; A61B 5/0261;**
**A61B 5/445; A61B 5/489**

(54) **PROCÉDÉ D ANALYSE D'UN ÉCHANTILLON PAR MESURE RÉSOLUE EN TEMPS DE L INTENSITÉ DE PHOTONS RÉTRODIFFUSÉS**

VERFAHREN ZUR ANALYSE EINER PROBE DURCH ZEITLICH AUFGELÖSTE MESSUNG DER INTENSITÄT VON RÜCKGESTREUTEN PHOTONEN

METHOD FOR ANALYSING A SAMPLE BY RESOLUTE MEASUREMENT OVER TIME OF THE INTENSITY OF BACKSCATTERED PHOTONS

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **29.12.2020 FR 2014215**

(43) Date de publication de la demande:
**06.07.2022 Bulletin 2022/27**

(73) Titulaire: **Commissariat à l'Energie Atomique et aux Energies Alternatives**
**75015 Paris (FR)**

(72) Inventeurs:
• **PLANAT-CHRETIEN, Anne**
**38054 Grenoble cedex 09 (FR)**
• **BERGER, Michel**
**38054 Grenoble cedex 09 (FR)**

(74) Mandataire: **INNOV-GROUP**
**310, avenue Berthelot**
**69372 Lyon Cedex 08 (FR)**

(56) Documents cités:
**EP-A1- 3 338 632 WO-A1-01/54580**

• ZUCCHELLI LUCIA ET AL: "Method for the discrimination of superficial and deep absorption variations by time domain fNIRS References and links | BIOMEDICAL OPTICS EXPRESS 2893", OPT. LETT. TRENDS NEUROSCI. PHYS. MED. BIOL. PHYS. MED. BIOL. PHYS. MED. BIOL. APPL. OPT. BIOMED. OPT. EXPRESS J. BIOMED. OPT. APPL. OPT. PHYS. MED. BIOL. PHYS. MED. BIOL.. D. CONTINI OPT. EXPRESS PHY , vol. 170528017025, no. 12 20 novembre 2013 (2013-11-20), pages 1766-1768, XP055838308, DOI: 10.1364/BOE.4.002893 Extrait de l'Internet: URL:https://www.ncbi.nlm.nih.gov/pmc/artic les/PMC3862167/pdf/2893.pdf
• Steinbrink J ET AL: "Physics in Medicine & Biology Determining changes in NIR absorption using a layered model of the human head", PHYSICS IN MEDICINE AND BIOLOGY Phys. Med. Biol, 1 janvier 2001 (2001-01-01), pages 879-896, XP055838830, Extrait de l'Internet: URL:https://iopscience.iop.org/article/10. 1088/0031-9155/46/3/320/pdf [extrait le 2021-09-07]
• MICHAEL S. WEINGARTEN ET AL: "Correlation of near infrared absorption and diffuse reflectance spectroscopy scattering with tissue neovascularization and collagen concentration in a diabetic rat wound healing model", WOUND REPAIR AND REGENERATION, vol. 16, no. 2, 1 mars 2008 (2008-03-01), pages 234-242, XP055088447, ISSN: 1067-1927, DOI: 10.1111/j.1524-475X.2008.00364.x

EP 4 023 147 B1

**Description**

**DOMAINE TECHNIQUE**

Le domaine technique de l'invention est l'application de mesures optiques au suivi de la vascularisation d'un lambeau sous-cutané, ayant été enfoui sous la peau d'une personne dans le cadre d'une chirurgie réparatrice.

**ART ANTERIEUR**

Dans le domaine de la chirurgie plastique reconstructrice, lorsqu'une perte de substance cutanée n'est pas suturable, ou ne peut pas cicatriser, ou nécessite un apport de matière, une technique chirurgicale possible est d'utiliser un lambeau pédiculé. Un tel lambeau est un fragment de peau et de tissu cellulaire sous-cutané, prélevé sur une autre partie du corps, et présentant une vascularisation autonome, passant par un pédicule. Au cours de la chirurgie de reconstruction et dans le cas d'une mastectomie par exemple, le lambeau est enfoui sous la peau d'origine pour reconstruire le volume du sein. Le pédicule est alors raccordé à une veine et une artère, de façon à permettre une irrigation du lambeau.

Ce type d'intervention suppose un suivi régulier de l'oxygénation du lambeau en post opératoire. Une vascularisation défaillante du lambeau enfoui, par exemple lors d'une thrombose, peut en effet avoir des conséquences graves, par exemple la survenue d'une nécrose suite à un manque d'oxygénation. Une telle situation nécessite généralement une intervention chirurgicale dans les plus brefs délais. Cependant la surveillance de la bonne oxygénation du lambeau est difficile du fait de sa non accessibilité.

On sait que les mesures optiques peuvent permettre, dans certaines conditions, un suivi de l'oxygénation de tissus superficiels. La spectroscopie de réflectance diffuse, usuellement désignée par l'acronyme DRS (Diffuse Reflectance Spectroscopy), est une technique d'analyse non destructive permettant d'estimer des propriétés de propagation de la lumière dans un milieu analysé. Cette technique est par exemple décrite dans EP2762064 ou EP3054282 ou EP3054281 ou EP3311138. Elle consiste à illuminer le milieu par un faisceau de lumière incident, et à détecter des photons rétrodiffusés par le milieu analysé, à distance du faisceau incident. Il est fréquent que la détection soit effectuée à différentes longueurs d'onde et ou à plusieurs distances du faisceau incident, de façon à obtenir des propriétés spectrales de propagation de la lumière dans le milieu analysé.

Les propriétés de propagation de la lumière comprennent généralement des propriétés d'absorption et/ou des propriétés de diffusion de la lumière. Il s'agit notamment de coefficients d'absorption ou de diffusion, ces derniers représentant respectivement des probabilités d'absorption et de diffusion d'un photon par unité de longueur. L'estimation des propriétés de propagation de la lumière, à certaines longueurs d'onde, permet une estimation d'une concentration d'analytes dans le milieu analysé. Ainsi, la DRS peut être utilisée pour estimer un taux d'oxygénation de tissus superficiels, par une comparaison entre une quantité d'oxyhémoglobine et une quantité de hémoglobine totale (oxyhémogobline + désoxyhémoglobine).

La spectroscopie de réflectance résolue en temps (TRS : Time-Resolved Reflectance Spectroscopy) prend en compte un aspect temporel de la mesure. Le milieu analysé est illuminé par un faisceau lumineux impulsionnel, de faible durée, typiquement comprise entre 10 et 100 ps. On détecte, à distance de ce dernier, les photons rétrodiffusés par le milieu, et on en détermine une distribution temporelle des photons détectés. La distribution temporelle permet de discriminer les photons détectés entre des photons prompts, correspondant aux premiers instants de la distribution temporelle, et des photons retardés, correspondant aux derniers instants de la distribution temporelle. Lors de l'interprétation des mesures, on prend en compte le fait que plus un photon est retardé, plus il est probable qu'il ait traversé des couches profondes du milieu. Une des applications de la TRS est l'analyse de milieux dans lesquels les propriétés de propagation de la lumière sont susceptibles de varier selon la profondeur, et en particulier entre des couches superficielles et des couches profondes. Des exemples d'application sont donnés dans les publications suivantes :

- Selb. J et al. «Improved sensitivity to cerebral hemodynamics during brain activation with a time-gated optical system: Analytical model and experimental validation », Journal of Biomerical Optics, 10(1), 2007.
- Contini, D et al. "Novel method for depth-resolved brain functional imaging by time-domain NIRS", Proc. SPIE 6629, Diffuse Optical imaging of Tissue, 662908. 12 July 2007.
- Zucchelli L, Contini D, Re R, Torricelli A, Spinelli L. « Method for the discrimination of superficial and deep absorption variations by time domain fNIRS. » Biomed Opt Express. 2013;4(12):2893-2910. Published 2013 Nov 20. doi:10.1364/BOE.4.002893.

Ces publications décrivent des exemples d'application de la TRS à des milieux diffusants formés de différentes couches superposées, présentant des propriétés de propagation de la lumière différentes les unes des autres.

Lorsque le milieu n'est pas homogène, et comporte plusieurs couches superposées, les photons retardés traversent chaque couche superposée. Le signal résultant des photons retardés dépend à la fois des propriétés optiques des couches superficielles et des couches profondes. Afin de caractériser correctement les couches profondes, la contribution des couches superficielles au signal détecté doit être prise en compte.

Les inventeurs proposent une méthode, basée sur la TRS, permettant un suivi post-opératoire de la vascularisation d'un lambeau. La méthode est non invasive. Elle peut permettre une estimation de la concentration de l'hémoglobine ou de l'oxyhémoglobine dans le lambeau.

## EXPOSE DE L'INVENTION

Un premier objet de l'invention est un procédé de suivi de la vascularisation d'un tissu biologique entre au moins un instant de mesure et un instant de référence, le tissu biologique étant délimité par une surface, le tissu biologique comportant une couche superficielle et une couche profonde, la couche superficielle s'étendant entre la surface du tissu et la couche profonde, le procédé comportant :

- a) illumination du tissu biologique par une source de lumière impulsionnelle, la source de lumière étant disposée en regard de la surface du tissu biologique, la source de lumière émettant un faisceau d'illumination formant une zone d'illumination à la surface du tissu biologique, le faisceau d'illumination étant émis simultanément ou successivement dans deux longueurs d'onde d'illumination différentes l'une de l'autre, chaque longueur d'onde s'étendant dans une bande spectrale d'absorption de l'oxyhémoglobine et/ou de la désoxyhémoglobine ;
- b) à chaque longueur d'onde, détection de photons rétrodiffusés par le tissu biologique, après s'être propagés dans le tissu biologique, par un photodétecteur, les photons rétrodiffusés détectés émanant d'une zone de détection à la surface du tissu biologique ;
- c ) à chaque longueur d'onde, obtention d'une distribution temporelle, représentant un nombre de photons détectés par le photodétecteur en fonction du temps, la distribution temporelle s'étendant selon une durée, à partir d'un instant initial ;

les étapes a) à c) étant effectuées à l'instant de référence et à l'instant de mesure ou chaque instant de mesure ; le procédé étant caractérisé en ce qu'il comporte, à l'instant de mesure ou chaque instant de mesure et à l'instant de référence :

- d) segmentation de la durée de chaque distribution temporelle en intervalles de détection, à chaque intervalle de détection étant associé un rang, le rang étant d'autant plus élevé que l'intervalle de détection est éloigné de l'instant initial de la distribution temporelle, chaque intervalle de détection étant associé à une couche élémentaire s'étendant à une profondeur dans le tissu biologique ;
- e) pour chaque couche élémentaire, à partir de chaque distribution temporelle résultant de c), établies à l'instant de mesure et à l'instant de référence, détermination d'intensités temporelles, correspondant à un nombre de photons détectés durant chaque intervalle de détection;

et en ce qu'il comporte, à l'instant de mesure ou à chaque instant de mesure

- f) pour chaque intervalle de détection, calcul d'un ratio entre l'intensité temporelle à l'instant de mesure et l'intensité temporelle à l'instant de référence ;
- g) à partir des ratios résultant de f), établis pour chaque longueur d'onde, calcul d'une grandeur d'intérêt pour chaque couche élémentaire respectivement associée à chaque intervalle de détection, la grandeur d'intérêt étant représentative d'une variation de la concentration d'hémoglobine, dans ladite couche élémentaire, entre l'instant de mesure et l'instant de référence ;
- h) pour chaque couche élémentaire, comparaison de la grandeur d'intérêt, calculée lors de g), avec une grandeur d'intérêt de base, calculée pour une couche élémentaire de base, la couche élémentaire de base étant une des couches élémentaires du tissu biologique, de façon à obtenir, pour chaque couche élémentaire, une grandeur d'intérêt différentielle ;
- i) détermination d'une évolution, selon la profondeur du tissu biologique, des grandeurs d'intérêt différentielles.

Avantageusement

- les étapes a) à h) sont mises en oeuvre à différents instants de mesure ;
- l'étape i) comporte une formation d'un profil, pour chaque couche de élémentaire, chaque profil représentant une

EP 4 023 147 B1

variation de la grandeur d'intérêt différentielle, dans ladite couche élémentaire, en fonction de l'instant de mesure ;
- l'étape i) comporte un calcul d'un critère de corrélation entre différents profils, correspondant respectivement à différentes couches élémentaires, le critère de corrélation traduisant une corrélation entre les différents profils;
- de telle sorte que la survenue de l'occlusion veineuse ou artérielle est détectée lorsque de part et d'autre d'une couche élémentaire, le critère de corrélation franchit une valeur seuil prédéterminée.

Le procédé peut comporter, suite à i) :

- j) détermination d'une couche élémentaire d'interface, choisie parmi les différentes couches élémentaires, la couche élémentaire d'interface étant une couche de part et d'autre de laquelle le critère de corrélation franchit la valeur seuil, la couche élémentaire d'interface étant considérée comme formant une interface entre la couche superficielle et la couche profonde, la couche élémentaire d'interface étant associée à un intervalle temporel d'interface ;
- k) à l'instant de référence et à un instant de mesure, à partir de chaque distribution temporelle résultant de c) établie à l'instant de mesure, calcul :

  • d'une première intensité temporelle, comportant une contribution, à la distribution temporelle, de photons détectés antérieurement à l'intervalle temporel d'interface, lesdits photons étant considérés comme s'étant propagés uniquement dans la couche superficielle ;
  • d'une deuxième intensité temporelle ; comportant une contribution à la distribution de photons détectés postérieurement à l'intervalle temporel d'interface, lesdits photons étant considérés comme s'étant propagés dans la couche superficielle et dans la couche profonde

- l) calcul d'un premier ratio, à partir des premières intensités temporelles respectivement déterminées à l'instant de référence et à l'instant de mesure et calcul d'un deuxième ratio, à partir des deuxièmes intensités temporelles respectivement déterminées à l'instant de référence et à l'instant de mesure ;
- m) estimation des variations des concentrations d'oxyhémoglobine et de désoxyhémoglobine ou de grandeurs proportionnelles auxdites variations, dans la couche profonde, entre l'instant de mesure et l'instant de référence, à partir des ratios résultant de l) ;

Le procédé peut comporter :

- o) estimation des variations des concentrations d'oxyhémoglobine et de désoxyhémoglobine, ou de grandeurs proportionnelles auxdites variations, dans la couche superficielle entre l'instant de mesure et l'instant de référence, à partir des ratios résultant de m).

Selon une possibilité, dans l'étape h), la couche de base est :

- une couche élémentaire prédéterminée ;
- ou une couche élémentaire de rang différent, par exemple inférieur, de la couche élémentaire considéré, l'écart entre le rang de la couche élémentaire de base et la couche élémentaire considéré étant constant.

Selon une possibilité, la source de lumière est une source laser.
Un deuxième objet de l'invention est un dispositif, destiné à détecter une survenue d'une occlusion veineuse ou artérielle dans un tissu biologique, entre au moins un instant de mesure et un instant de référence, le tissu biologique étant délimité par une surface, le dispositif comportant :

- une source de lumière impulsionnelle, la source de lumière étant disposée en regard de la surface du tissu biologique, la source de lumière étant configurée pour émettre un faisceau d'illumination, formant une zone d'illumination, à la surface du tissu biologique, la source de lumière étant configurée pour émettre simultanément ou successivement le faisceau d'illumination dans deux longueurs d'onde d'illumination différentes l'une de l'autre, chaque longueur d'onde s'étendant dans une bande spectrale d'absorption de l'oxyhémoglobine et/ou de la désoxyhémoglobine ;
- un photodétecteur, configuré pour détecter des photons rétrodiffusés par le tissu biologique, après s'être propagés dans le tissu biologique, les photons rétrodiffusés émanant d'une zone de détection à la surface du tissu biologique ;
- une carte de comptage, destinée à établir une distribution temporelle, représentant un nombre de photons détectés par le photodétecteur en fonction du temps, à chaque longueur d'onde ;
- une unité de traitement, programmée pour mettre en oeuvre les étapes d) à i) d'un procédé selon le premier objet de l'invention à partir des distributions temporelles établies par la carte de comptage à chaque longueur d'onde, à l'instant de mesure ou à chaque instant de mesure.

4

L'invention sera mieux comprise à la lecture de l'exposé des exemples de réalisation présentés, dans la suite de la description, en lien avec les figures listées ci-dessous.

## FIGURES

La figure 1A schématise un dispositif permettant une mise en oeuvre de l'invention.

La figure 1B montre une zone d'illumination et une zone de détection formées à la surface d'un échantillon.

La figure 1C schématise une impulsion d'illumination et une distribution temporelle de photons rétrodiffusés par l'échantillon, et détectés par un photodétecteur, les photons émanant de l'échantillon à travers la zone de détection.

La figure 1D illustre une configuration selon laquelle le dispositif comporte plusieurs fibres d'illumination et de détection.

La figure 2A illustre une distribution temporelle de photons détectés segmentée en différents intervalles temporels.

La figure 2B schématise une subdivision de l'échantillon en différentes couches, chaque couche correspondant à une profondeur atteinte par des photons détectés durant un intervalle temporel.

La figure 3 montre les principales étapes permettant une mise en oeuvre de l'invention.

La figure 4A montre une segmentation de l'échantillon en deux couches, séparées par une interface s'étendant à une profondeur de séparation par rapport à la surface de l'échantillon.

La figure 4B montre une distribution temporelle de photons détectés segmentée en deux intervalles temporels, chaque intervalle temporel correspondant respectivement à une première couche et à une deuxième couche de l'échantillon.

La figure 5A schématise un échantillon utilisé lors d'essais expérimentaux.

La figure 5B représente un profil, montrant une évolution, à plusieurs instants de mesure successifs, d'une grandeur d'intérêt, proportionnelle à une concentration différentielle apparente dans différentes profondeurs de l'échantillon. Une concentration différentielle apparente est une comparaison entre des concentrations apparentes respectivement à un instant de mesure et à un instant de référence. La figure 5B correspond à l'évolution de la quantité d'oxyhémoglobine, au cours d'une occlusion artérielle.

La figure 5C représente un profil, montrant une évolution, à plusieurs instants de mesure successifs, de la grandeur d'intérêt décrite en lien avec la figure 5B. La figure 5C correspond à l'évolution de la quantité de désoxyhémoglobine, au cours de la même occlusion artérielle.

La figure 5D représente un profil montrant une évolution, à plusieurs instants de mesure successifs, d'une comparaison entre les grandeurs d'intérêts représentées sur la 5B entre deux couches élémentaires de rang $w$ et $w$-3, pour différents ordres w successifs

La figure 5E représente un profil montrant une évolution, à plusieurs instants de mesure successifs, d'une comparaison entre les grandeurs d'intérêts représentées 5C entre deux couches élémentaires de rang w et $w$-3, pour différents ordres w successifs La figure 6A montre des coefficients de corrélation entre les profils représentés sur la figure 5D.

La figure 6B montre des coefficients de corrélation entre les profils représentés sur la figure 5E.

La figure 6C résulte du produit des coefficients de corrélation obtenues en 6A et 6B.

La figure 7A représente des estimations, selon le temps, de concentrations d'oxyhémoglobine et de désoxyhémoglobine résultant respectivement d'une mise en oeuvre d'une méthode de spectrométrie de réflectance diffuse résolue en temps sans prise en compte de la couche superficielle (courbes a et b respectivement) et d'une mise en oeuvre d'une méthode de spectrométrie de réflectance diffuse non résolue en temps, selon l'art antérieur (courbes c et d). La figure 7A est représentative d'une occlusion artérielle.

La figure 7B représente des concentrations d'oxyhémoglobine (courbe a) et de désoxyhémoglobine (courbe b) illustrées dans la figure 7A, corrigée selon la mise en oeuvre de l'invention ainsi qu'un lissage temporel des concentrations estimées à différents instants de mesure.

La figure 8A montre une estimation des concentrations d'oxyhémoglobine (courbe a) et de désoxyhémoglobine (courbe b) au cours d'une occlusion artérielle selon la mise en oeuvre de l'invention, ainsi que le résultat de mesures résultant d'une sonde Licox. (courbe c).

La figure 8B est équivalente à la figure 8A, dans le cas d'une occlusion veineuse.

## EXPOSE DE MODES DE REALISATION PARTICULIERS

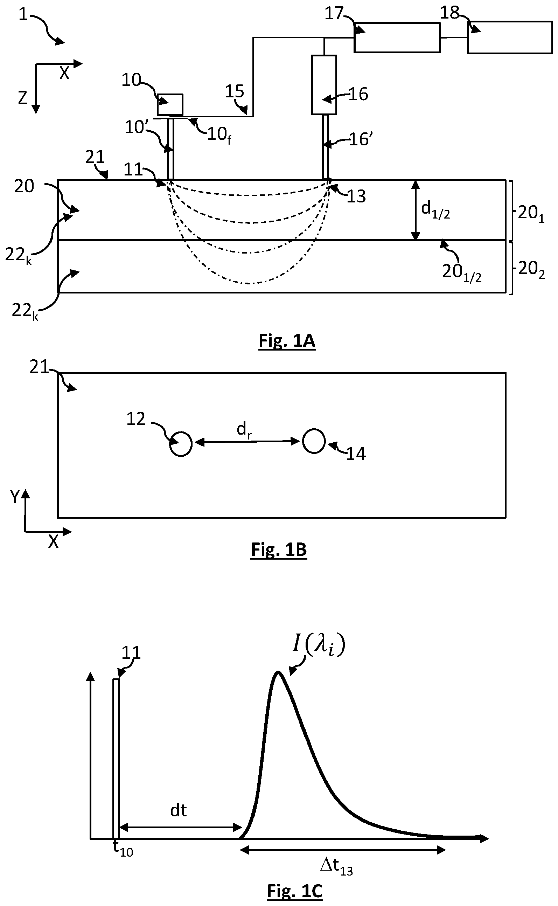

La figure 1A représente un dispositif 1 permettant un suivi de la vascularisation d'un lambeau enfoui sous un une couche de peau. L'objectif est de déterminer une survenue d'une occlusion veineuse ou artérielle, en particulier durant les premières heures ou les premiers jours après la chirurgie.

Le dispositif comporte une source de lumière 10 impulsionnelle. Elle est configurée pour émettre un faisceau lumineux 11 impulsionnel, la durée de chaque impulsion étant typiquement entre 1 ps et 100 ps. La fréquence d'impulsion peut être par exemple comprise entre 1 et 100 MHz.

Le faisceau lumineux 11, dit faisceau d'illumination, se propage en direction d'un échantillon de tissu biologique 20 à analyser. L'échantillon 20 est délimité par une surface 21. L'intersection du faisceau d'illumination 11 et de la surface 21 de l'échantillon forme une zone d'illumination 12, spatialement délimitée. La zone d'illumination est de préférence ponctuelle : elle est par exemple inscrite dans un cercle de diamètre inférieur à 1mm, voire inférieur à quelques centaines de $\mu$m, par exemple 100 $\mu$m ou 1 mm. La zone élémentaire d'illumination 12 est représentée sur la figure 1B.

La source de lumière peut être disposée au contact du tissu biologique 20 ou à distance de ce dernier. De préférence, la source de lumière est une source de lumière laser femtoseconde. Dans l'exemple représenté, la source de lumière est disposée à distance de l'échantillon. Le faisceau lumineux 11 est transporté jusqu'à la surface 21 de l'échantillon par une fibre optique d'illumination 10'.

Les photons formant le faisceau d'illumination 11 se propagent dans le tissu biologique à analyser. Le tissu biologique 20 est formé d'un milieu diffusant, susceptible d'absorber les photons, les propriétés de propagation des photons dans le milieu dépendant notamment de propriétés d'absorption ou de diffusion dans le milieu. De façon usuelle, les propriétés d'absorption peuvent être quantifiées par un coefficient d'absorption linéaire $\mu_a(\lambda)$. De façon connue, le coefficient d'absorption linéaire quantifie une probabilité d'absorption par le milieu par unité de longueur, à la longueur d'onde $\lambda$. Il est usuellement exprimé en cm$^{-1}$. Les propriétés de diffusion peuvent être quantifiées par un coefficient de diffusion $\mu_s(\lambda)$ ou un coefficient de diffusion réduit $\mu_s'(\lambda)$, qui quantifient une probabilité de diffusion par le milieu par unité de longueur, à la longueur d'onde $\lambda$. Il est usuellement exprimé en cm$^{-1}$.

Le dispositif comporte un photodétecteur 16. Il peut par exemple s'agir d'un tube photomultiplicateur, de type tube photomultiplicateur hybride, usuellement désigné par l'acronyme HPM (Hybrid Photo Multiplier - Photomultiplicateur Hybride). Les photons détectés par le photodétecteur 16 émanent d'une zone élémentaire de détection 14 à la surface 21 de l'échantillon 20. La zone élémentaire de détection 14 est de préférence ponctuelle, en étant, à l'instar de la zone élémentaire d'illumination 12, inscrite dans un diamètre inférieur à 1 mm voire à 250 $\mu$m. La zone élémentaire de détection 14 est confondue ou séparée de la zone élémentaire d'illumination 12. La distance entre la zone élémentaire d'illumination 12 et la zone élémentaire de détection 14 est une distance de rétrodiffusion $d_r$. Elle peut être de quelques centimètres ou de l'ordre du centimètre, voire peut être inférieure à 1 cm.

Les photons 13 émanant de l'échantillon, à travers la zone élémentaire de détection 14, sont des photons rétrodiffusés par l'échantillon selon la distance de rétrodiffusion $d_r$. Le photodétecteur 16 peut être disposé au contact ou à distance de l'échantillon 20. Dans l'exemple représenté, une fibre optique de détection 16' transporte les photons rétrodiffusés 13 entre la zone élémentaire de détection 14 et le photodétecteur 16.

La source de lumière 10 peut être monochromatique ou polychromatique. Dans l'exemple représenté, la source de lumière est un laser femtoseconde de type super continuum (supercontinu) émettant des impulsions lumineuses selon une fréquence de 80 MHz. Une sélection de la longueur d'onde d'illumination $\lambda_i$ est effectuée par un filtre optique 10$_f$. Dans l'exemple décrit, le filtre 10$_f$ est une roue à filtres comportant différents filtres interférentiels passe-bande permettant une sélection de la longueur d'onde d'illumination selon l'une des valeurs suivantes : 750 nm, 800 nm ou 850 nm. Le filtre 10$_f$ peut également comporter un filtrage passe-haut, en complément des filtres interférentielles passe-bande.

Dans le mode de réalisation de l'invention, on utilise deux longueurs d'onde d'illumination $\lambda_1$ et $\lambda_2$ qui correspondent à des longueurs d'onde d'absorption de la désoxyhémoglobine et de l'oxyhémoglobine. Par exemple, $\lambda_1 = 750$ nm et $\lambda_2 = 850$ nm. La notation $\lambda_i$ désigne indifféremment une longueur d'onde d'illumination, qu'il s'agisse de $\lambda_1$ ou de $\lambda_2$.

Le photodétecteur 16 est relié à une carte électronique de comptage 17 de type TCSPC (Time-Correlated Single Photon Counting - Comptage de photon unique résolu en temps). Ce type de carte électronique de comptage est bien connu dans le domaine des mesures optiques résolues en temps. La carte de comptage établit une distribution temporelle $I(\lambda_i)$ des photons détectés par le photodétecteur 16. La distribution temporelle est établie statistiquement suite à une multitude d'impulsions lumineuses émises successivement par la source de lumière 10. La distribution temporelle $I(\lambda_i)$ correspond à un histogramme représentant un nombre de photons rétrodiffusés 13 détectés (axe des ordonnées) en fonction du temps (axe des abscisses).

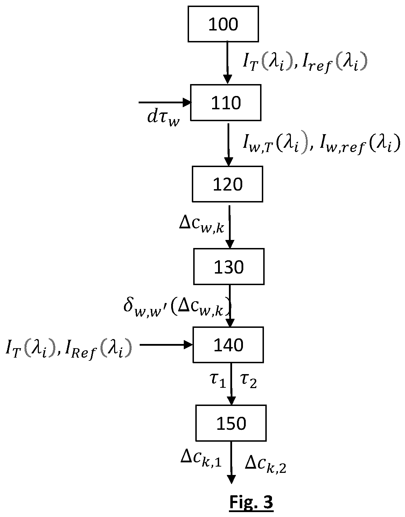

La carte de comptage 17 est reliée à une unité de traitement 18. L'unité de traitement 18 comporte un microprocesseur programmé pour mettre en oeuvre les étapes décrites par la suite, en lien avec la figure 3, à partir des distributions temporelles $I(\lambda_i)$ établies par la carte de comptage 17. Le dispositif peut comporter un ou plusieurs atténuateurs optiques, chaque atténuateur optique étant usuellement désigné « densité optique ». L'homme du métier adapte l'atténuation en fonction de la puissance de la source de lumière et de la sensibilité du photodétecteur.

La carte de comptage 17 est synchronisée par une ligne de synchronisation 15, qui détecte chaque impulsion lumineuse émise par la source de lumière 10. Cela permet de déterminer un intervalle temporel dt entre l'émission et la détection de chaque photon détecté par le photodétecteur 16. Sur la figure 1C, on a représenté une impulsion lumineuse de photons incidents 11, survenant à un instant d'illumination $t_{10}$, et une distribution temporelle de photons rétrodiffusés 13, cette dernière s'étendant selon un intervalle temporel $\Delta t_{13}$.

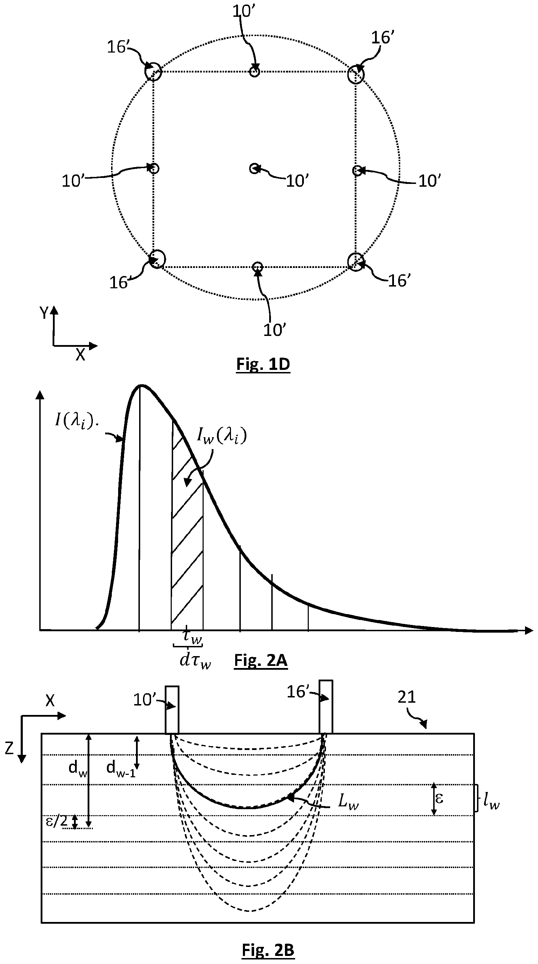

En multipliant le nombre de zones élémentaires d'illumination 12 et de zones élémentaires de détection 14, il est possible d'adresser séquentiellement différentes distances de rétrodiffusion $d_r$. La figure 1D représente une configuration selon laquelle la source de lumière 10 est reliée à cinq fibres optiques d'illumination 10', par l'intermédiaire d'un commutateur optique (switch optique). Les fibres optiques d'illumination 10' sont réparties respectivement au centre et au milieu des côtés d'un carré de côté 12 mm. Selon cette configuration, le dispositif 1 comporte quatre fibres optiques de détection 16', respectivement reliées à quatre photodétecteurs 16 différents. Les fibres optiques de détection 16' sont respectivement disposées sur les quatre coins du carré de côté 12 mm précédemment décrit. Une telle configuration permet d'adresser des distances de rétrodiffusion respectivement égales à 6 mm, 8.5 mm et 13.4 mm.

La puissance de la source de lumière incidente est monitorée par un prélèvement de la lumière d'excitation assuré par une fibre optique 15'.

Sur la figure 2A, on a schématisé une segmentation d'une distribution temporelle $I(\lambda_i)$ entre différentes fenêtres temporelles centrées correspondant à des intervalles temporels $d\tau_w$. Chaque intervalle temporel $d\tau_w$ s'étend autour d'un instant de détection $\tau_w$. La segmentation de la distribution temporelle permet d'estimer un nombre de photons détectés durant chaque intervalle temporel $d\tau_w$, ce qui correspond une intensité temporelle $I_w(\lambda_i)$ pour chaque intervalle temporel $d\tau_w$. L'indice w est un entier correspondant au rang w de chaque intervalle temporel avec $1 < w < N_w$, $N_w$ étant le nombre d'intervalles temporels considérés. La durée de chaque intervalle temporel $d\tau_w$ peut être inférieure à une nanoseconde, en étant par exemple de l'ordre de quelques dizaines de ps.

Cette segmentation temporelle peut être faite, à partir de la distribution temporelle, par application d'une fenêtre temporelle en créneau de largeur fixe, les fenêtres temporelles étant décalées les unes par rapport aux autres. Les fenêtres temporelles peuvent se superposer l'une à l'autre. Par exemple, la largeur de chaque fenêtre temporelle peut être de 0.2 ns, le décalage de deux fenêtres temporelles consécutives s'élevant à 0.1 ns. Selon une autre possibilité, les fenêtres temporelles peuvent être définies en appliquant des transformées de Mellin Laplace de différents ordres à la distribution temporelle $I(\lambda_i)$, comme décrit dans EP2605002 ou dans les publications :

- L. Hervé et al. « Time-domaine diffuse optical tomography processing by suing the Mellin-Laplace transform », Applied Optics 51(25), 5978-5988 (2012) ;
- J. Zaouaoui « Chromophore décomposition in multispectral time-resolved diffuse optical tomography », Biomédical optics Express 8(10), 4772 (2017).

De façon connue, à chaque intervalle temporel $d\tau_w$ correspond une profondeur $d_w$ susceptible d'être atteinte par les photons dans chaque couche de l'échantillon 20. Plus le rang w de l'instant $\tau_w$ est élevé, plus les photons détectés peuvent être considérés comme ayant atteint une profondeur $d_w$ importante dans l'échantillon. Comme on peut le voir sur la figure 2B, La profondeur $d_w$ est déterminée par rapport à la surface 21 de l'échantillon, comportant la zone élémentaire d'illumination 12. Ainsi, à chaque intervalle temporel $d\tau_w$ centré sur l'instant de détection $\tau_w$ peut être associé à une couche élémentaire $I_w$ de rang w s'étendant à une profondeur $d_w \pm \varepsilon/2$, où $\varepsilon$ est l'épaisseur de la couche élémentaire, qui dépend de la largeur de l'intervalle temporel $d\tau_w$ considéré.

Comme précédemment évoqué, le dispositif est configuré pour permettre une détection d'une occlusion veineuse ou artérielle dans un lambeau enfoui sous une couche de peau. Le dispositif est appliqué contre la surface 21 d'un tissu biologique 20, ce dernier comportant une couche superficielle $20_1$, de peau, et une couche profonde $20_2$. La couche superficielle est interposée entre la surface du tissu et la couche profonde. Sous l'effet d'une occlusion artérielle ou veineuse, la concentration d'hémoglobine dans la couche profonde peut subir une variation progressive par rapport à la couche superficielle, entre l'instant de référence et l'instant de mesure. Par hémoglobine, il est entendu les deux états de l'hémoglobine, à savoir l'oxyhémoglobine et la désoxyhémoglobine. Deux longueurs d'onde d'illumination sont successivement utilisées qui correspondent à des longueurs d'onde absorbée par l'oxyhémoglobine et la désoxyhémoglobine.

Le terme couche est à distinguer du terme « couche élémentaire ». Une couche correspond à une partie macroscopique de l'échantillon dans laquelle les propriétés optiques sont considérées comme homogènes. Une couche élémentaire correspond à une « microcouche », dont l'épaisseur et la profondeur dépendent de la segmentation temporelle effectuée sur la distribution temporelle.

Comme représenté sur la figure 1A, la couche superficielle et la couche profonde sont séparées l'une de l'autre par une interface $20_{1/2}$. L'interface est située à une profondeur d'interface $d_{1/2}$ par rapport à la surface 21 de l'échantillon. Cependant, la profondeur d'interface n'est pas connue. La profondeur d'interface $d_{1/2}$ peut être située à quelques mm, par exemple 3 mm ou 5 mm, à quelques cm, par exemple 1 ou 2 cm, par rapport à la surface 21 de l'échantillon.

Un aspect important de l'invention est d'estimer expérimentalement des instants $\tau_w$ de la distribution temporelle, respectivement associés à la couche superficielle et à la couche profonde. Il s'agit de discriminer précisément :

- les photons prompts, considérés comme s'étant propagés à travers la couche superficielle $20_1$, sans s'être propagés à travers la couche profonde $20_2$, ou de façon marginale : sur la figure 1A, la trajectoire des photons prompts est matérialisée par des lignes en tirets.

- les photons retardés, considérés comme s'étant propagés à travers la couche superficielle $20_1$ et à travers la couche profonde $20_2$ : sur la figure 1A, la trajectoire des photons retardés est matérialisée par des lignes en tirets mixtes.

La figure 3 schématise les principales étapes du procédé, dans un cas d'application pour lequel on cherche à déterminer la survenue d'une occlusion veineuse ou artérielle dans la couche profonde du tissu biologique examiné. Il s'agit des étapes 100 à 130. Le procédé peut également permettre une identification d'une occlusion détectée : il s'agit de savoir si l'occlusion est veineuse ou artérielle. Cela correspond aux étapes 140 et 150. Au cours des étapes 140 et 150, on cherche à estimer une variation de la concentration $\Delta c_k$ d'hémoglobine $22_k$, par rapport à une concentration de référence $c_{k,ref}$. L'hémoglobine peut être de l'oxyhémoglobine ($k = 1$) ou la désoxyhémoglobine ($k=2$). La concentration de référence correspond à une concentration de l'hémoglobine à un instant de référence $T_{ref}$. On se place dans un cas de figure selon lequel la concentration $c_k$ de l'hémoglobine est susceptible de varier dans le temps, et de manière différente dans les deux couches. Un aspect important de l'invention est de pouvoir déterminer une variation de concentration d'oxyhémoglobine et de désoxyhémoglobine (ou au moins une grandeur proportionnelle à chaque variation) dans la couche profonde, sans qu'il soit nécessaire de déterminer la variation de concentration d'oxyhémoglobine et de désoxyhémoglobine dans la couche superficielle.

Le dispositif permet ainsi d'effectuer des mesures de réflectance optique résolues en temps peuvent permettre un suivi de l'oxygénation du lambeau, de façon non invasive.

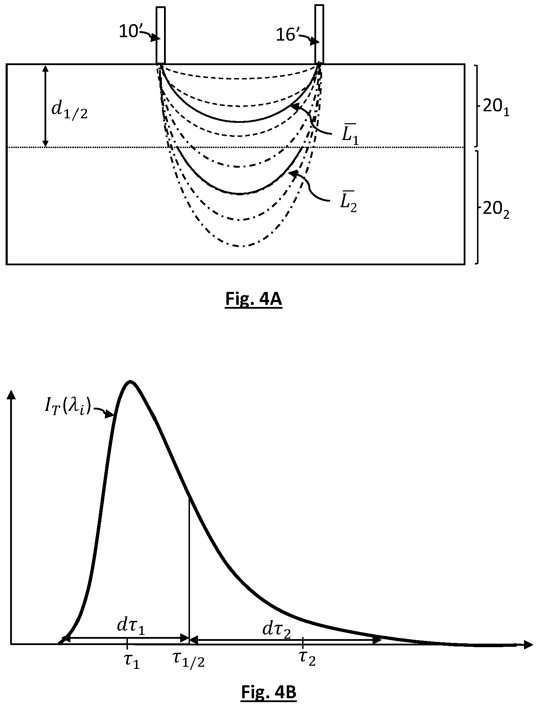

Le principe de l'invention est schématisé sur les figures 4A et 4B. Sur la figure 4A, on a représenté l'échantillon, avec les photons respectivement issus de la couche superficielle $20_1$ et de la couche profonde $20_2$. Lorsqu'une occlusion veineuse ou artérielle survient dans la couche profonde, la réponse de la couche profonde à l'égard du faisceau d'illumination varie. Cela se traduit par une variation du signal rétrodiffusé. Ce signal est sensible aux variations de la couche profonde, mais aussi de la couche superficielle : si la couche superficielle évolue (du fait de la chirurgie par exemple), alors le signal rétrodiffusé va traduire l'évolution des deux couches ; pour être à même de détecter et identifier correctement l'occlusion survenue en couche profonde, il faut corriger ce signal de la contribution de la couche superficielle : c'est l'objet de l'invention.

Un premier objectif de l'invention est décrit en lien avec les étapes 100 à 130 décrites ci-après : il s'agit de détecter la survenue d'une occlusion veineuse ou artérielle dans la couche profonde $20_2$, sachant que la profondeur de l'interface $20_{1/2}$ entre les deux couches n'est pas connue.

Un deuxième objectif, complémentaire au premier, et optionnel, est d'effectuer un suivi de la concentration d'oxyhémoglobine ou d'oxyhémoglobine dans la couche profonde $20_2$. Cela correspond aux étapes 140 et 150. Pour cela, on détermine expérimentalement un premier intervalle temporel $d\tau_1$, et un deuxième intervalle temporel $d\tau_2$, qui correspondent respectivement aux contributions des photons « prompts » et des photons retardés à la distribution temporelle $I_T$.

### Détection de la survenue d'une occlusion

### Etape 100 : acquisition

Au cours de cette étape, l'échantillon 20 est illuminé de façon impulsionnelle. A partir de photons rétrodiffusés 13 détectés par le photodétecteur 16 après chaque impulsion, on constitue une distribution temporelle $I(\lambda_i)$ à chaque longueur d'onde d'émission $\lambda$.

L'étape 100 peut être effectuée séquentiellement ou simultanément pour plusieurs longueurs d'onde $\lambda_i$, de façon à obtenir, pour chaque longueur d'onde, une distribution temporelle $I(\lambda_i)$. L'indice i désigne le rang de chaque longueur d'onde.

La distribution temporelle $I(\lambda_i)$ représente une évolution d'une intensité des photons rétrodiffusés détectés par le photodétecteur 16, en fonction du temps Selon un mode de réalisation, l'intensité peut être normalisée par l'intensité $I_{10}$ émise par la source de lumière 10. Dans ce cas, une fibre, dite de retour d'excitation, s'étend entre la source de lumière 10 et un photodétecteur 16. Une telle normalisation permet de prendre en compte les fluctuations de la source de lumière.

Dans la suite, le terme intensité désigne indifféremment une intensité ou une intensité normalisée par l'intensité $I_{10}$ du faisceau d'illumination.

Dans cet exemple, l'étape 100 est mise en oeuvre à différentes instants de mesure T, de façon à permettre un contrôle régulier de l'échantillon examiné. Elle est notamment mise en oeuvre à un instant de référence $T_{ref}$ lors de laquelle l'échantillon examiné est considéré comme dans un état de référence. Lorsque l'échantillon est un tissu cutané ayant fait l'objet d'une reconstruction chirurgicale par un lambeau, l'instant de référence peut correspondre à une mesure effectuée immédiatement suite à l'intervention chirurgicale, l'échantillon étant considéré comme correctement oxygéné.

Au cours de l'instant de référence $T_{ref}$, on obtient une distribution temporelle de référence $I_{ref}(\lambda_i)$ à chaque longueur d'onde $\lambda_i$. Au cours de chaque instant de mesure $T$, la distribution temporelle d'intensité est notée $I_T(\lambda_i)$.

<u>Etape 110</u> : détermination d'intensités temporelles.

Au cours de cette étape, chaque distribution temporelle $I_T(\lambda_i)$, fait l'objet d'une décomposition en fenêtres temporelles, de façon à obtenir une intensité temporelle $I_{w,T}(\lambda_i)$ de photons rétrodiffusés détectés dans l'intervalle temporel $d\tau_w$ à la longueur d'onde $\lambda_i$. Le terme intensité temporelle désigne une intensité détectée dans chaque intervalle temporel $d\tau_w$.

Comme précédemment indiqué, la décomposition temporelle peut être effectuée par une application d'une transformée de Mellin-Laplace à la distribution temporelle $I_T(\lambda_i)$, ou par l'application de fenêtres temporelles en créneaux (ou d'autres types de fenêtres temporelles). A l'issue de cette étape, on dispose d'intensités temporelles $I_{w,T}(\lambda_i)$ de la distribution temporelle $I_T(\lambda_i)$, chaque intensité temporelle $I_{w,T}(\lambda_i)$ étant associée à un intervalle temporel $d\tau_w$ centré sur un instant de détection $\tau_w$. Le référentiel temporel des mesures est donné par les instants de mesure $T$, espacés de quelques minutes à plusieurs heures. Chaque instant de mesure est décomposé en intensité temporelles $I_{w,T}(\lambda_i)$ pour des instants successifs $\tau_w$, $\tau_{w+1}$ rapprochés d'une durée de quelques dizaines de ps ou centaines de picosecondes (ps), par exemple 200 ps. A chaque instant de détection $\tau_w$ correspond un intervalle temporel de détection $d\tau_w$ s'étendant de part et d'autre de l'instant de détection $\tau_w$.

<u>Etape 120</u> : Explicitation de la concentration différentielle.

Au cours de cette étape, on détermine, à partir de chaque intensité temporelle résultant de l'étape 110, une concentration $c_{w,k}$ de l'hémoglobine $22_k$ dans chaque couche élémentaire $l_w$ traversée par les photons détectés dans la fenêtre temporelle de rang $w$, s'étendant autour de l'instant de détection $\tau_w$.

Les inventeurs considèrent que selon une première approximation,

$$\frac{I_{w,T}(\lambda_i)}{I_{w,ref}(\lambda_i)} = e^{-(\mu_{a,w,T}(\lambda_i) - \mu_{a,w,ref}(\lambda_i))L_w} = e^{-(\Delta\mu_{a,w,T}(\lambda_i))L_w} \quad (1)$$

$\Delta\mu_{a,w,T}(\lambda_i)$ correspond à une variation du coefficient d'absorption, à la longueur d'onde $\lambda_i$, déterminée d'après la fenêtre temporelle de rang $w$, entre l'instant de mesure $T$ et l'instant de référence $T_{ref}$ :

$$\Delta\mu_{a,w,T}(\lambda_i) = \mu_{a,w,T}(\lambda_i) - \mu_{a,w,ref}(\lambda_i) \quad (1')$$

$\mu_{\alpha,w,T}(\lambda_i)$ et $\mu_{\alpha,w,ref}(\lambda_i)$ sont respectivement un coefficient d'absorption à l'instant de mesure $T$ et à l'instant de référence $T_{ref}$, pour la longueur d'onde $\lambda_i$.

$L_w$ est le libre parcours moyen des photons sélectionnés sur $\tau_w$. En première approximation, $L_w$ est indépendant de la longueur d'onde ; il peut être estimé par $L_w = \gamma\tau_w$, $\gamma$ étant un coefficient de proportionnalité.

Ainsi, l'étape 120 étape comporte une comparaison, dans chaque fenêtre temporelle de rang $w$, entre une intensité $I_{w,T}(\lambda_i)$ et une intensité de référence $I_{w,ref}(\lambda_i)$, établies, pour la même fenêtre temporelle, respectivement à partir des distributions temporelles $I_T(\lambda_i)$ et $I_{ref}(\lambda_i)$.

A partir de (1), on peut écrire :

$$\ln\left(\frac{I_{w,T}(\lambda_i)}{I_{w,ref}(\lambda_i)}\right) = -\left(\Delta\mu_{a,w,T}(\lambda_i)\right)L_w \quad (2)$$

Or

$$\mu_{a,w,T}(\lambda_i) = \sum_k c_{w,k,T}\varepsilon_k(\lambda_i)L_w \quad (3)$$

$c_{w,k,T}$ est la concentration de l'hémoglobine (k = 1 : oxyhémoglobine ; k=2 : désoxyhémoglobine) lors de l'instant de mesure $T$ ;

$\varepsilon_k(\lambda)$ est un coefficient d'absorption linéaire l'hémoglobine pour une concentration unitaire (unité $cm^2/g$ lorsque la concentration est exprimée en $g/cm^3$), également désigné coefficient d'extinction. $\varepsilon_k(\lambda)$ est une donnée connue, disponible dans des bases de données.

Lors de l'instant de référence,

$$\mu_{a,w,ref}(\lambda_i) = \sum_k c_{w,k,ref}\varepsilon_k(\lambda_i)L_w \quad (4)$$

$c_{w,k,ref}$ est la concentration de l'hémoglobine lors de l'instant de référence $T_{ref}$ ;

La combinaison de (2), (3) et (4) donne :

$$-\ln\left(\frac{I_{w,T}(\lambda_i)}{I_{w,ref}(\lambda_i)}\right) = \sum_k \Delta c_{w,k,T}\varepsilon_k(\lambda_i)L_w \quad (5)$$

avec :

$$\Delta c_{w,k,T} = c_{w,k,T} - c_{w,k,ref} \quad (6)$$

$\Delta c_{w,k,T}$ correspond à une variation de la concentration l'hémoglobine, correspondant à la fenêtre temporelle de rang w, entre l'instant de mesure $T$ et l'instant de référence $T_{ref}$. Par la suite, $\Delta c_{w,k,T}$ est désignée par le terme «concentration différentielle apparente».

Lorsque deux longueurs d'onde Â, , $\lambda_2$ sont mises en oeuvre, l'expression (5) peut être présentée sous forme matricielle :

$$\begin{bmatrix} -\ln\left(\frac{I_{w,T}}{I_{w,ref}}\right)(\lambda_1) \\ -\ln\left(\frac{I_{w,T}}{I_{w,ref}}\right)(\lambda_2) \end{bmatrix} = L_w \begin{bmatrix} \varepsilon_1(\lambda_1) & \varepsilon_2(\lambda_1) \\ \varepsilon_1(\lambda_2) & \varepsilon_2(\lambda_2) \end{bmatrix} \begin{bmatrix} \Delta c_{w,1} \\ \Delta c_{w,2} \end{bmatrix} \quad (7)$$

où :

Il est à noter que le recours aux expressions (1), (2), (5) et (7) repose sur une hypothèse simplificatrice, selon laquelle la concentration d'hémoglobine, à une couche élémentaire $l_w$, d'ordre w, est estimée sans prendre en compte des inhomogénéités du milieu, à des couches élémentaires d'ordre inférieur $l_1$ ... $.l_{w-1}$. Or, comme précédemment évoqué, l'échantillon peut présenter des inhomogénéités de la concentration d'hémoglobine en fonction de la profondeur. L'hypothèse simplificatrice utilisée au cours de cette étape revient à considérer que la concentration de l'hémoglobine est homogène entre la surface de l'échantillon et la couche élémentaire $l_w$. Ainsi, chaque valeur $\Delta c_{w,k}$ peut être assimilée à une concentration différentielle apparente, le terme « apparente » désignant le fait qu'elle est estimée sur la base d'une hypothèse d'homogénéité de la concentration de l'hémoglobine dans l'échantillon, jusqu'à la profondeur $d_w$.

Une telle simplification est justifiée par le fait que chaque concentration différentielle est utilisée, dans les étapes 130 et 140, pour effectuer une segmentation de l'échantillon entre une première couche $20_1$ et une deuxième couche $20_2$, la concentration de l'hémoglobine dans chaque couche ainsi segmentée étant considérée comme homogène.

On considère à présent, pour chaque couche élémentaire $l_w$, une grandeur d'intérêt $\Delta\zeta_{w,k}$, telle que :

$$\Delta\zeta_{w,k} = L_w\Delta c_{w,k} \quad (8)$$

La grandeur d'intérêt $\Delta\zeta_{w,k}$ est proportionnelle à une variation de la concentration différentielle apparente $\Delta c_{w,k}$ de la couche élémentaire $l_w$ entre l'instant de référence et l'instant de mesure. La grandeur $\zeta_{w,k}$ peut être considérée comme une concentration surfacique apparente. Elle correspond à la concentration apparente $c_{w,k}$ multipliée par le libre parcours moyen des photons $L_w$. La grandeur $\zeta_{w,k}$ est équivalente à une concentration surfacique apparente, obtenue comme la contribution du milieu entre la surface à la couche élémentaire d'ordre w.

Chaque grandeur d'intérêt ($\Delta\zeta_{w,1}$ pour l'oxyhémoglobine et $\Delta\zeta_{w,2}$ pour la désoxyhémoglobine) peut être calculée directement à partir de $I_{w,T}$ et de $I_{w,ref}$ en résolvant l'équation (10), qui résulte de l'équation (7) :

$$\begin{bmatrix} -\ln\left(\dfrac{I_{w,T}}{I_{w,ref}}\right)(\lambda_1) \\[2ex] -\ln\left(\dfrac{I_{w,T}}{I_{w,ref}}\right)(\lambda_2) \end{bmatrix} = \begin{bmatrix} \varepsilon_1(\lambda_1) & \varepsilon_2(\lambda_1) \\ \varepsilon_1(\lambda_2) & \varepsilon_2(\lambda_2) \end{bmatrix} \begin{bmatrix} \Delta\zeta_{w,1} \\ \Delta\zeta_{w,2} \end{bmatrix} \quad (10)$$

Etape 130 : comparaison des grandeurs d'intérêt $\Delta\zeta_{w,k}$ résultant de l'étape 120.

L'invention consiste à comparer ces grandeurs d'intérêt $\Delta\zeta_{w,k}$ obtenues en considérant respectivement deux couches élémentaires $I_w$ de rangs différents.

Pour chaque couche élémentaire $I_w$, on obtient une grandeur d'intérêt différentielle $\delta_{w,w'}(\Delta\zeta_{w,k})$, correspondant à une différence entre :

-   une grandeur d'intérêt, obtenue pour une couche élémentaire de base $I_{w'}$, de rang w' (avec w' < w)
-   une grandeur d'intérêt, obtenue pour la couche élémentaire $I_w$ de rang w.

Dans cet exemple, on établit une comparaison $\delta_{w,w'}(\Delta\zeta_{w,k})$ telle que

$$\delta_{w,w'}(\Delta\zeta_{w,k}) = \Delta\zeta_{w,k} - \Delta\zeta_{w',k} = L_w\Delta c_{w,k} - L_{w'}\Delta c_{w',k} \quad (11)$$

La couche élémentaire de base $I_{w'}$ peut être de rang fixe, par exemple w'= 1, correspondant aux photons les plus prompts de la distribution, auquel cas :

$$\delta_{w,w'}(\Delta\zeta_{w,k}) = \Delta\zeta_{w,k} - \Delta\zeta_{w'=1,k} \quad (12)$$

La couche élémentaire de base $L_{w'}$ peut être telle que w' = w - 1, auquel cas

$$\delta_{w,w'}(\Delta\zeta_{w,k}) = \Delta\zeta_{w,k} - \Delta\zeta_{w-1,k} \quad (13)$$

Lorsque les intensités temporelles $I_{w,T}$, $I_{w,ref}$ sont obtenues en appliquant une transformée de Mellin Laplace, la couche temporelle de base $d\tau_{w'}$ peut être tel que w' = w - 3.

Il est préférable que le rang de la couche temporelle de base w' soit fixe, ou obtenu par un décalage fixe par rapport au rang de la couche élémentaire considérée, par exemple w' = w - 3 ou w' = w - 1

Pour chaque couche élémentaire $I_w$, la quantité $\delta_{w,w'}(\Delta\zeta_{w,k})$ représente une variation, en fonction de w, de la grandeur d'intérêt $\Delta\zeta_{w,k}$ entre la couche élémentaire $I_w$ et la couche élémentaire de base $I_{w'}$.

A partir des intensités temporelles résultant de l'étape 110, on peut calculer, pour différents instants de mesure T, une grandeur d'intérêt différentielle $\delta_{w,w'}(\Delta\zeta_{w,k})$ pour différentes couches élémentaires $\zeta_{w,k}$ d'ordre w.

L'évolution, en fonction de w, de $\delta_{w,w'}(\Delta\zeta_{w,k})$ permet d'identifier d'éventuelles discontinuités survenant, au cours du temps, dans l'échantillon. Lors de la survenue d'une occlusion, veineuse ou artérielle, dans la couche profonde, une discontinuité apparaît entre $\delta_{w,w'}(\Delta\zeta_{w,k})$, pour les rangs w correspondant à la couche superficielle, et $\delta_{w,w'}(\Delta\zeta_{w,k})$ pour les rangs w correspondant à la couche profonde. Autrement dit, lorsqu'une occlusion survient, on observe une décorrélation entre les valeurs de $\delta_{w,w'}(\Delta\zeta_{w,k})$ obtenues pour les couches élémentaires $I_w$ situées respectivement dans la couche superficielle $20_1$ et dans la couche profonde $20_2$.

Lorsque les étapes 100 à 130 sont mises en oeuvre à différents instants de mesure, on peut obtenir, pour chaque couche élémentaire de rang w, un profil traçant l'évolution temporelle de la grandeur d'intérêt différentielle $\delta_{w,w'}(\Delta\zeta_{w,k})$. En cas de survenue d'une occlusion artérielle ou veineuse, la décorrélation entre la couche superficielle et la couche profonde est plus aisée à détecter sur la base d'un tel profil.

La décorrélation entre les valeurs de $\delta_{w,w'}(\Delta\zeta_{w,k})$ obtenues respectivement entre la couche superficielle et la couche profonde peut être déterminée visuellement, par un utilisateur du dispositif. De préférence, un indicateur de corrélation est calculé, qui permet de quantifier une corrélation entre deux valeurs de $\delta_{w,w'}(\Delta\zeta_{w,k})$ pour deux couches de rang w différents. Il peut s'agir d'un coefficient de corrélation $r^2$. Plus la valeur de l'indicateur de corrélation est élevée, plus les valeurs de $\delta_{w,w'}(\Delta\zeta_{w,k})$, pour deux couches de rang w différents, sont proches. En cas de survenue d'une occlusion, l'indicateur de corrélation entre deux couches élémentaires $I_w$ de rangs w différents franchit un seuil prédéterminé, en dessous duquel on considère qu'il y a une décorrélation.

Essais expérimentaux

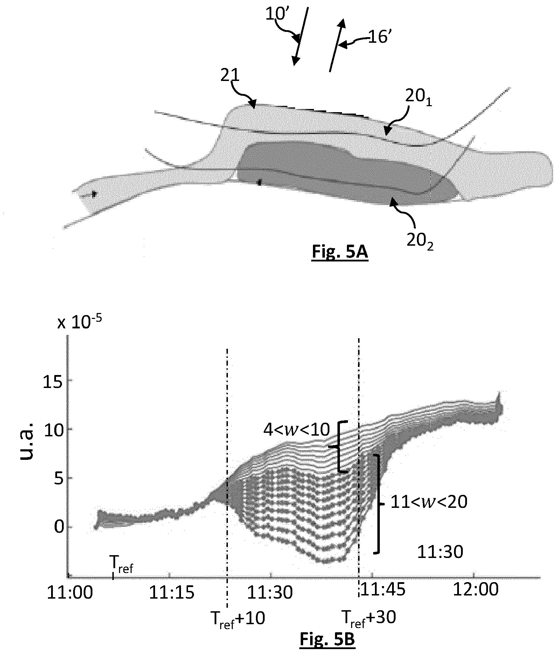

On a mis en oeuvre le procédé précédemment décrit, en utilisant un échantillon comportant un lambeau de cochon implanté en dessous d'une couche de peau, tel que représenté sur la figure 5A. La couche de peau formait couche superficielle $20_1$ tandis que le lambeau formait une couche profonde $20_2$. Le lambeau était relié à une artère et à une veine. Un clamp permettait de bloquer l'irrigation soit de l'artère (occlusion artérielle), soit de la veine (occlusion veineuse). Le dispositif mettait en oeuvre une source de lumière et des photodétecteurs fibrés, comme représenté sur la figure 1D. Les paramètres expérimentaux étaient les suivants :

- épaisseur de la couche superficielle (peau + graisse) : environ 1 cm ;
- épaisseur du lambeau : environ 1 cm ;
- source de lumière utilisée : laser supercontinuum fibré - Fianium WI-SC-480-10 fréquence d'impulsion 80 MHz ;
- photodétecteurs : photomultiplicateur hybride - Becker-Hickl HPM 100-50. Un filtre passe haut - fréquence de coupure à 690 nm - était associé à chaque photodétecteur. Référence filtre : HLP690 - Melles Griot.
- carte électronique TCSPC : Becker-Hickl TCSPC-SPCM 134
- sélection de la longueur d'onde : premier filtrage par deux filtres passe haut Thorlabs FEL 1000 et FGL 570, couplés à une roue à filtres comportant des filtres centrés sur les longueurs d'onde de 750 nm, 800 nm et 850 nm - Largeur à mi-hauteur 10 nm.
- Un atténuateur de lumière était disposé entre chaque fibre d'illumination 10' et la source de lumière : densité optique variable entre 0 et 4.
- Distance de rétrodiffusion : 13 mm

Le protocole d'acquisition a été décomposé comme suit

- entre $T_{ref}$ et $T_{ref}$ + 20 min (minutes) : le lambeau est irrigué normalement par la veine et l'artère, $t_0$ correspondant à une série d'acquisitions ;
- entre $T_{ref}$ + 20 min et $T_{ref}$ + 40 min : clampage de l'artère (occlusion artérielle) ou de la veine (occlusion veineuse) : le lambeau n'est plus irrigué correctement;
- entre $T_{ref}$ + 40 min et $T_{ref}$ + 60 min : le clampage est arrêté. Irrigation normale du lambeau.

Entre $T_{ref}$ et $T_{ref}$ + 60 min, le procédé décrit dans les étapes 100 à 130 a été suivi, en différents instants de mesure $T$, chaque instant de mesure étant espacée l'un de l'autre de 45 secondes.

Lors de l'étape 120, on a extrait différentes intensités temporelles $I_w$ en appliquant des fonctions de segmentation temporelles en appliquant une transformée de Mellin Laplace. Lors de cette étape, on a considéré un nombre total $W$ d'intervalles temporels égal à 20.

Dans l'exemple donné par les figures 5B à 5E, il s'agit d'une occlusion artérielle.

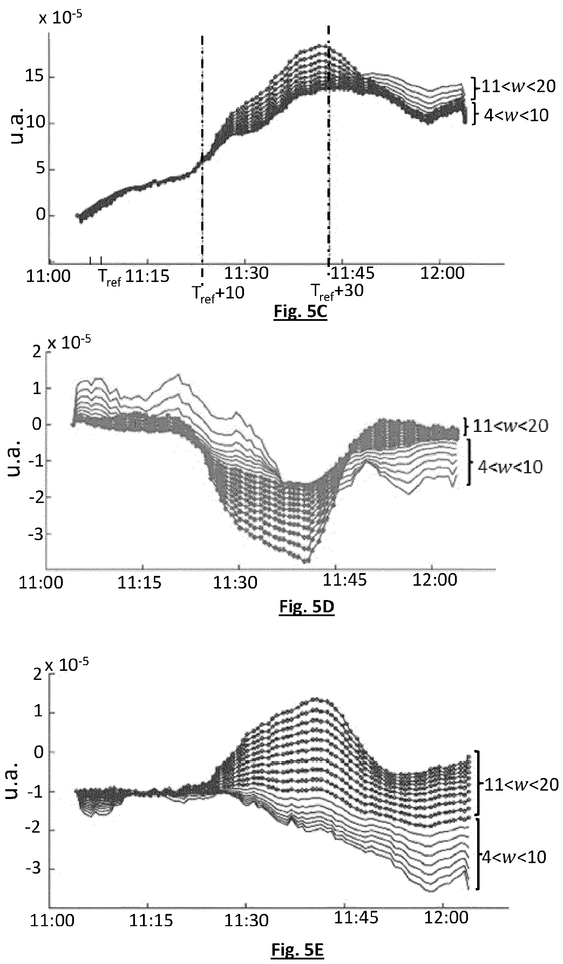

Les figures 5B et 5D représentent respectivement, à chaque instant de mesure (axe des abscisses) des valeurs de $\Delta\zeta_{w,k=1}$ et de $\delta_{w,w'}(\Delta\zeta_{w,k=1})$ pour les valeurs de $w$ comprises entre 4 et 20. Lorsque k=1, il est rappelé qu'on adresse l'oxyhémoglobine.

Les figures 5C et 5E représentent respectivement, à chaque instant de mesure (axe des abscisses) des valeurs de $\Delta\zeta_{w,k=2}$ et de $\delta_{w,w'}(\Delta\zeta_{w,k=2})$ pour les valeurs de w comprises entre 4 et 20. Lorsque k=2, il est rappelé qu'on adresse la désoxyhémoglobine.

Les courbes 5B à 5E correspondent à des profils temporels traçant les évolutions temporelles respective de $\Delta\zeta_{w,k=1}$, $\delta_{w,w'}(\Delta\zeta_{w,1})$, $\Delta\zeta_{w,k=2}$, $\delta_{w,w'}(\Delta\zeta_{w,2})$.

Sur les figure 5B et 5C, avant le clampage ($T_{ref}$ < T < $T_{ref}$ + 20 min), les courbes sont proches les unes des autres, ce qui traduit une certaine homogénéité. Durant le clampage, ($T_{ref}$ + 20 < T < $T_{ref}$ + 40 min), les courbes se séparent les unes des autres, ce qui traduit l'apparition d'une inhomogénéité de la concentration d'oxyhémoglobine (courbe 5B) et de désoxyhémoglobine (courbe 5C). Sans surprise, les grandeurs d'intérêt $\Delta\zeta_{w,k}$, qui sont proportionnelles à une variation de la concentration d'oxyhémoglobine (ou de désoxyhémoglobine) entre l'instant de mesure et l'instant de référence, traduisent, au cours du clampage artériel dans cet exemple, une diminution de la concentration d'oxyhémoglobine et une légère augmentation de la concentration de désoxyhémoglobine.

Pour définir s'il y a ou non occlusion, on cherche à mettre en évidence une différence d'évolution dans le tissu d'intérêt. Pour cela, on s'intéresse en particulier aux grandeurs d'intérêt différentielles $\delta_{w,w'}(\Delta\zeta_{w,k})$. Pour chaque analyte (oxyhémoglobine et désoxyhémoglobine), les valeurs sont faiblement dispersées avant l'occlusion. L'occlusion engendre une dispersion plus importante. Sur la base des figures 5D et 5E, on note un comportement similaire pour les profils lorsque w < 11 : on considère alors que les mesures sont représentatives de la couche superficielle. Lorsque w ≥ 11, les mesures sont représentatives de la couche profonde. Les grandeurs d'intérêt différentielles permettent de déterminer une interface de part et d'autre de laquelle ces grandeurs ne sont plus corrélées lorsqu'une occlusion survient.

Les profils temporels représentés sur les figures 5B à 5E permettent une détection de la survenue d'une occlusion artérielle à partir des grandeurs d'intérêt différentielles $\delta_{w,w'}(\Delta\zeta_{w,k})$.

La détection d'une survenue d'une occlusion peut être effectuée visuellement, ou en calculant un indicateur de corrélation entre les valeurs de $\delta_{w,w'}(\Delta\zeta_{w,k})$ entre des couches élémentaires d'ordres w différents.

Chaque grandeur d'intérêt différentielle est représentative d'une variation spatiotemporelle d'une concentration d'oxyhémoglobine ou de désoxyhémoglobine. La variation temporelle correspond au terme $\Delta\zeta_{w,k}$, qui représente une variation de $\zeta_{w,k}$ entre deux instants différents (instant de mesure et instant de référence). La variation spatiale provient de la différence entre les grandeurs $\Delta\zeta_{w,k}$ entre la couche de rang w et la couche de rang w'.

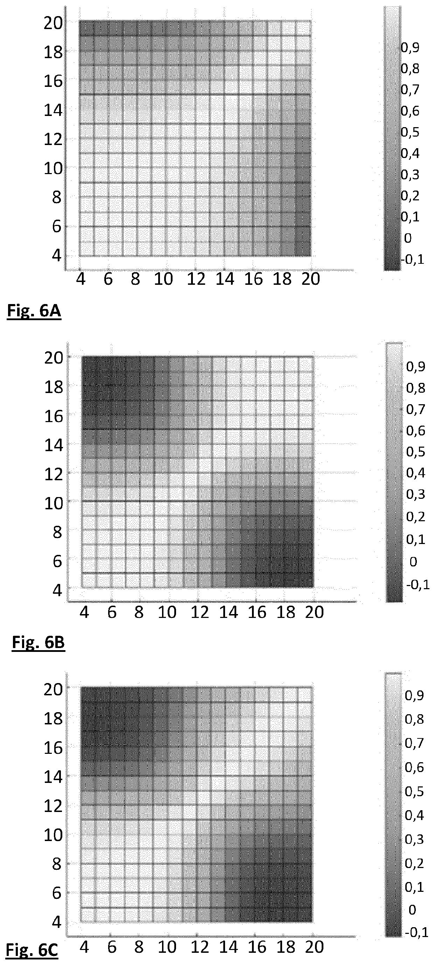

Les figures 6A à 6C représentent respectivement des coefficients de corrélation calculés respectivement :

- à partir des profils de la figure 5D (oxyhémoglobine) ;
- à partir des profils de la figure 5E (desoxyhémoglobine) ;
- en combinant les profils de la figure 5D et 5E.

La figure 6A montre un indicateur de corrélation entre des profils de la figure 5D correspondant à des rangs w différents. Sur cette figure, l'axe des abscisses et l'axe des ordonnées correspondent à un rang w. Le niveau de gris correspond au coefficient de corrélation. De même la figure 6B montre un indicateur de corrélation entre des profils de la figure 5E correspondant à des rangs w différents.

La figure 6C est une synthèse figures 6A et 6B : elle représente le produit des coefficients de corrélation présentés en figure 6A et 6B. Sur cette figure, on observe une bonne corrélation des couches élémentaires 1 à 11, ainsi que des couches élémentaires 13 à 20.

Estimation de variations de concentrations d'hémoglobine entre l'instant de mesure et l'instant de référence.

On va décrire à présent des étapes optionnelles, qui permettent une analyse plus approfondie des tissus examinés. Après avoir suspecté la survenue d'une difficulté vasculaire, telle ubne occlusion, l'objectif est d'identifier le type d'inclusion suspecté, afin de déterminer s'il s'agit d'une occlusion veineuse ou artérielle.

Etape 140 : segmentation temporelle

La décorrélation entre les grandeurs d'intérêt ou les grandeurs d'intérêt différentielles, décrites dans l'étape 130 et sur les figures 5B à 5E, ainsi que 6A à 6C, permet de définir une couche élémentaire de rang $w_{1/2}$, de part et d'autre de laquelle les grandeurs d'intérêt, ou les grandeurs d'intérêt différentielles, ne sont pas corrélées. Une telle couche élémentaire correspond à une couche d'interface $I_{w1/2}$. Elle est considérée comme formant une interface entre la couche superficielle $20_1$ d'une part, et la couche profonde $20_2$ d'autre part. La couche d'interface $I_{w1/2}$ est associée à un intervalle temporel d'interface $d\tau_{1/2}$ : cf. figures 4A et 4B

A partir de l'intervalle temporel d'interface $d\tau_{1/2}$, il est possible d'estimer :

- un premier intervalle temporel $d\tau_1$, centré sur un instant de détection $\tau_1$ correspondant aux photons prompts considérés comme n'ayant pas traversé la couche profonde $20_2$;
- un deuxième intervalle temporel $d\tau_2$, centré sur un instant de détection $\tau_2$, correspondant aux photons retardés considérés comme ayant traversé la couche superficielle $20_1$ ainsi que la couche profonde $20_2$.

Etape 150 : estimation d'une variation de concentration d'hémoglobine dans la couche profonde $20_2$, entre l'instant de mesure et l'instant de référence.

Dans cette étape, on considère que l'on a deux couches : la couche $20_1$ (couche superficielle) et la couche $20_2$ (couche profonde). Sur les distributions temporelles $I_T(\lambda_i)$ et $I_{Ref}(\lambda_i)$, les contributions $I_{1,T}$, $I_{1,ref}$ de la couche $20_1$ et $I_{2,T}$, $I_{2,ref}$ de la couche $20_2$ correspondent respectivement :

- aux photons détectés durant le premier intervalle temporel $d\tau_1$,
- aux photons détectés durant l'intervalle temporel $d\tau_2$;

A partir des mesures $I_{1,T}, I_{1,ref}, I_{1,T}, I_{1,ref}$, on peut écrire :

$$\begin{bmatrix} -\ln\left(\frac{I_{1,T}}{I_{1,ref}}\right)(\lambda_1) \\ \vdots \\ -\ln\left(\frac{I_{1,T}}{I_{1,ref}}\right)(\lambda_2) \end{bmatrix} = \bar{L}_1 \begin{bmatrix} \varepsilon_1(\lambda_1) & \varepsilon_2(\lambda_1) \\ \varepsilon_1(\lambda_2) & \varepsilon_2(\lambda_2) \end{bmatrix} \begin{bmatrix} \Delta c_{1,1} \\ \Delta c_{1,2} \end{bmatrix} \quad (15)$$

$\bar{L}_1$ correspond au libre parcours moyen dans la couche $20_1$ : $\bar{L}_1$ correspond à une moyenne des libres parcours moyens des photons contribuant à $I_{1T}$ et $I_{1,ref}$. $\bar{L}_1$ est représenté sur la figure 4A. De même, $\bar{L}_2$ correspond au libre parcours moyen dans la couche $20_2$. $\bar{L}_2$ correspond à une moyenne des libres parcours moyens des photons contribuant à $I_{2,T}$ et $I_{2,ref}$.

En utilisant le fait que

$$\Delta\mu_{a,1}(\lambda_1)\bar{L}_1 + \Delta\mu_{a,2}(\lambda_1)\bar{L}_2 = -\ln\left(\frac{I_{2,T}(\lambda_1)}{I_{2,ref}(\lambda_1)}\right) \quad (16)$$

et

$$\Delta\mu_{a,1}(\lambda_2)\bar{L}_1 + \Delta\mu_{a,2}(\lambda_2)\bar{L}_2 = -\ln\left(\frac{I_{2,T}(\lambda_2)}{I_{2,ref}(\lambda_2)}\right) \quad (16')$$

il vient

$$\begin{bmatrix} -\ln\left(\frac{I_{2,T}}{I_{2,ref}}\right)(\lambda_1) + \ln\left(\frac{I_{1,T}}{I_{1,ref}}\right)(\lambda_1) \\ -\ln\left(\frac{I_{2,T}}{I_{2,ref}}\right)(\lambda_2) + \ln\left(\frac{I_{1,T}}{I_{1,ref}}\right)(\lambda_2) \end{bmatrix} = \bar{L}_2 \begin{bmatrix} \varepsilon_1(\lambda_1) & \varepsilon_2(\lambda_1) \\ \varepsilon_1(\lambda_2) & \varepsilon_2(\lambda_2) \end{bmatrix} \begin{bmatrix} \Delta c_{2,1} \\ \Delta c_{2,2} \end{bmatrix} (17)$$

Sachant que, la matrice $\begin{bmatrix} \varepsilon_1(\lambda_1) & \varepsilon_2(\lambda_1) \\ \varepsilon_1(\lambda_2) & \varepsilon_2(\lambda_2) \end{bmatrix}$ est connue et que les grandeurs $-\ln\left(\frac{I_{1,T}}{I_{1,ref}}\right)(\lambda_1)$ et $-\ln\left(\frac{I_{1,T}}{I_{1,ref}}\right)(\lambda_2)$, $-\ln\left(\frac{I_{1,T}}{I_{1,ref}}\right)(\lambda_2)$ et $-\ln\left(\frac{I_{1,T}}{I_{1,ref}}\right)(\lambda_2)$

résultent des distributions temporelles mesurées à l'instant de mesure et à l'instant de référence, à chaque longueur d'onde, on obtient $\bar{L}_2 \begin{bmatrix} \Delta c_{2,1} \\ \Delta c_{2,2} \end{bmatrix}$, c'est-à-dire des grandeurs $\bar{L}_2\Delta c_{2,1}$ et $\bar{L}_2\Delta c_{2,2}$, proportionnelles aux variations de concentrations $\Delta c_{2,1}$ et $\Delta c_{2,2}$ respectivement de l'oxyhémoglobine et de la désoxyhémoglobine dans la deuxième couche $20_2$.

Cela permet d'identifier le type d'occlusion, c'est-à-dire de déterminer s'il s'agit d'une occlusion veineuse ou artérielle.

Si l'on souhaite estimer les concentrations $\Delta c_{2,1}$ et $\Delta c_{2,2}$, le libre parcours moyen $\bar{L}_2$ peut être estimé.

Il est également possible d'estimer $\bar{L}_1\Delta c_{1,1}$ et $\bar{L}_1\Delta c_{1,2}$, dans la première couche, à partir de l'expression

$$\begin{bmatrix} -\ln\left(\frac{I_{1,T}}{I_{1,ref}}\right)(\lambda_1) \\ \vdots \\ -\ln\left(\frac{I_{1,T}}{I_{1,ref}}\right)(\lambda_2) \end{bmatrix} = \bar{L}_1 \begin{bmatrix} \varepsilon_1(\lambda_1) & \varepsilon_2(\lambda_1) \\ \varepsilon_1(\lambda_2) & \varepsilon_2(\lambda_2) \end{bmatrix} \begin{bmatrix} \Delta c_{1,1} \\ \Delta c_{1,2} \end{bmatrix} \quad (18)$$

A partir d'une estimation de $\overline{L}_1$, on peut obtenir $\Delta c_{1,1}$ et $\Delta c_{1,2}$.

Essais expérimentaux

A partir des figures 5B à 5E et 6A à 6C, on a estimé la couche d'interface de rang $w_{1/2}$ entre la première couche $20_1$ et la deuxième couche $20_2$. On a mis en oeuvre les étapes 140 et 150 de façon à estimer, à chaque instant de mesure, des grandeurs $\overline{L}_2\Delta c_{2,1}$ et $\overline{L}_2\Delta c_{2,2}$ respectivement proportionnelles aux variations de concentrations d'oxyhémoglobine et de désoxyhémoglobine entre chaque instant de mesure et l'instant de référence.

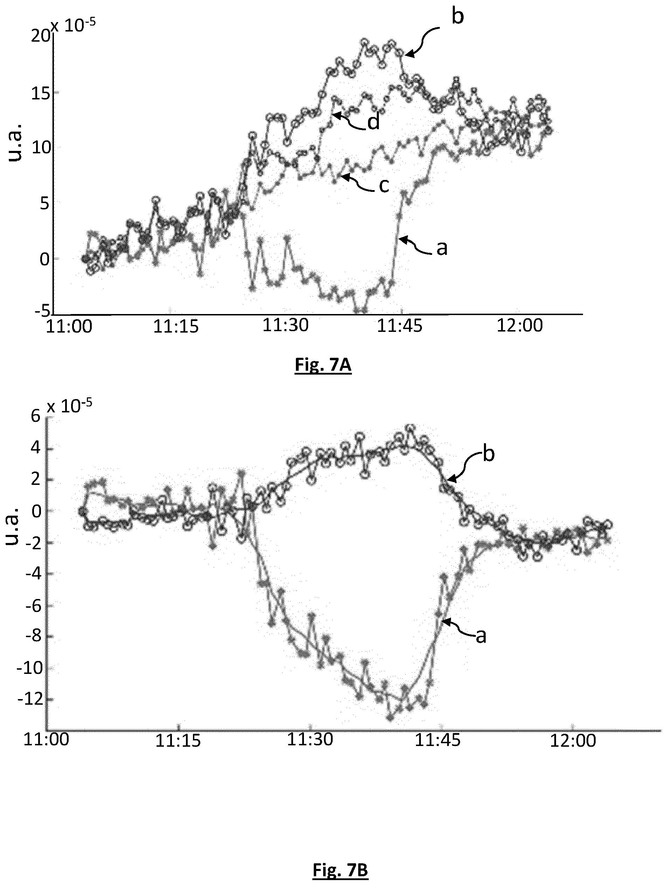

La figure 7A représente des estimations, selon le temps, de concentrations d'oxyhémoglobine et de désoxyhémoglobine résultant respectivement d'une mise en oeuvre d'une méthode de spectrométrie de réflectance diffuse résolue en temps sans prise en compte de la couche superficielle (courbes a et b respectivement) et d'une mise en oeuvre d'une méthode de spectrométrie de réflectance diffuse non résolue en temps, selon l'art antérieur (courbes c et d). La figure 7A est représentative d'une occlusion artérielle.

La figure 7B représente des variations de concentrations d'oxyhémoglobine (courbe a) et de désoxyhémoglobine (courbe b) illustrées dans la figure 7A, corrigée selon la mise en oeuvre de l'invention ainsi qu'un lissage temporel des concentrations estimées à différents instants de mesure. Sur la figure 7B, l'axe des ordonnées correspond aux variations de concentrations d'oxyhémoglobine et de désoxyhémoglobine, dans la couche $20_2$, multipliées par $\overline{L}_2$ : il s'agit de $\overline{L}_2\Delta c_{2,1}$ et $\overline{L}_2\Delta c_{2,2}$.

Lors des essais expérimentaux, des sondes Licox (marque déposée) ont été mises en oeuvre dans la couche superficielle et dans la couche profonde. Une sonde Licox, connue de l'homme du métier, permet de mesurer une pression partielle d'oxygène dans les tissus.

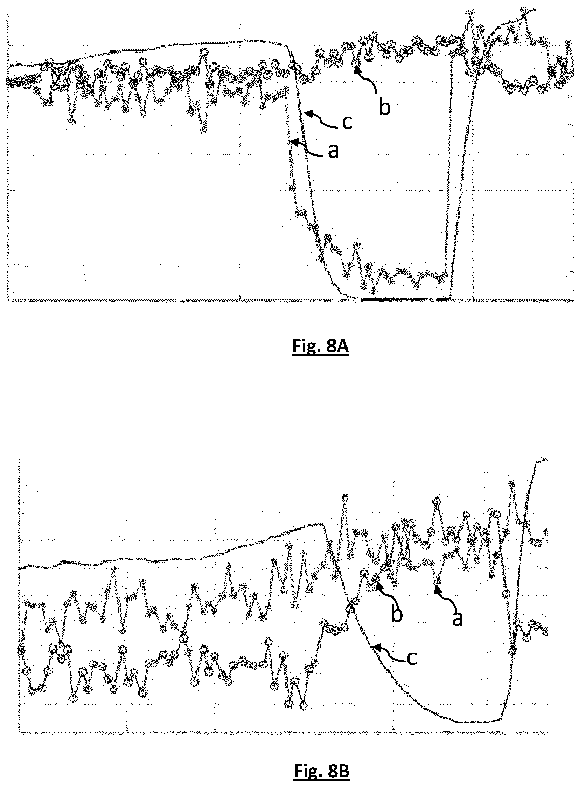

La figure 8A représente une estimation de la variation de concentration d'oxyhémoglobine (courbe a) et de désoxyhémoglobine (courbe b), chaque variation étant multipliée par $\overline{L}_2$, résultant de la mise en oeuvre des étapes 100 à 150, lors d'une occlusion artérielle. On a également représenté une courbe résultant de la sonde Licox (courbe c).

La figure 8B représente une estimation de la variation de concentration d'oxyhémoglobine (courbe a) et de désoxyhémoglobine (courbe b), chaque variation étant multipliée par $\overline{L}_2$, résultant de la mise en oeuvre des étapes 100 à 150, lors d'une occlusion veineuse. On a également représenté une courbe résultant de la sonde Licox (courbe c).

Une sonde Licox ne permet pas de remonter à une estimation d'une variation concentration. De plus, il s'agit d'une mesure invasive. La sonde Licox permet de détecter la survenue d'une occlusion, qu'il s'agisse d'une occlusion artérielle ou veineuse, sans pouvoir discriminer le type d'occlusion. On observe que les mesures selon l'invention, non invasives, sont cohérentes avec les mesures Licox pour détecter la survenue d'une occlusion. Elles permettent une estimation d'une variation de la concentration d'oxyhémogline et de désoxyhémoglobine par rapport à un instant de référence. On peut ainsi identifier le type d'occlusion : artérielle ou veineuse.

Les mesures telles qu'illustrées sur les figures 8A et 8B ont été effectuées sur quinze cochons différents : les occlusions artérielles ou veineuses ont été correctement identifiées dans chaque essai.

**Revendications**

1. Procédé de suivi de la vascularisation d'un tissu biologique entre au moins un instant de mesure (T) et un instant de référence $(T_{ref})$, le tissu biologique étant délimité par une surface (21), le tissu biologique comportant une couche superficielle ($20_1$) et une couche profonde ($20_2$), la couche superficielle s'étendant entre la surface du tissu et la couche profonde, le procédé comportant :

   - a) illumination du tissu biologique par une source de lumière impulsionnelle (10), la source de lumière étant disposée en regard de la surface du tissu biologique, la source de lumière émettant un faisceau d'illumination (11) formant une zone d'illumination (12) à la surface du tissu biologique, le faisceau d'illumination étant émis simultanément ou successivement dans deux longueurs d'onde d'illumination différentes l'une de l'autre, chaque longueur d'onde s'étendant dans une bande spectrale d'absorption de l'oxyhémoglobine et/ou de la désoxyhémoglobine ;
   - b) à chaque longueur d'onde, détection de photons rétrodiffusés par le tissu biologique, après s'être propagés dans le tissu biologique, par un photodétecteur (16), les photons rétrodiffusés détectés émanant d'une zone de détection (14) à la surface du tissu biologique ;
   - c) à chaque longueur d'onde, obtention d'une distribution temporelle $(I_T(\lambda_i), I_{Ref}(\lambda_i))$, représentant un nombre de photons détectés par le photodétecteur en fonction du temps, la distribution temporelle s'étendant selon une durée, à partir d'un instant initial ;

les étapes a) à c) étant effectuées à l'instant de référence et à l'instant de mesure ou chaque instant de mesure ; le procédé étant **caractérisé en ce qu'**il comporte, à l'instant de mesure ou chaque instant de mesure et à l'instant de référence :

- d) segmentation de la durée de chaque distribution temporelle en intervalles de détection ($d\tau_w$), à chaque intervalle de détection étant associé un rang (w), le rang étant d'autant plus élevé que l'intervalle de détection est éloigné de l'instant initial de la distribution temporelle, chaque intervalle de détection étant associé à une couche élémentaire ($l_w$) s'étendant à une profondeur ($d_w$) dans le tissu biologique ;
- e) pour chaque couche élémentaire ($l_w$), à partir de chaque distribution temporelle résultant de c), établies à l'instant de mesure et à l'instant de référence, détermination d'intensités temporelles ($I_{w,T}$, $I_{w,ref}$), correspondant à un nombre de photons détectés durant chaque intervalle de détection ($d\tau_w$) ;

et **en ce qu'**il comporte, à l'instant de mesure ou à chaque instant de mesure

- f) pour chaque intervalle de détection ($d\tau_w$), calcul d'un ratio entre l'intensité temporelle à l'instant de mesure et l'intensité temporelle à l'instant de référence ;
- g) à partir des ratios résultant de f), établis pour chaque longueur d'onde, calcul d'une grandeur d'intérêt ($\Delta\zeta_{w,k}$) pour chaque couche élémentaire respectivement associée à chaque intervalle de détection, la grandeur d'intérêt étant représentative d'une variation de la concentration d'hémoglobine, dans ladite couche élémentaire, entre l'instant de mesure et l'instant de référence ;
- h) pour chaque couche élémentaire ($l_w$), comparaison de la grandeur d'intérêt, calculée lors de g), avec une grandeur d'intérêt de base, calculée pour une couche élémentaire de base, la couche élémentaire de base étant une des couches élémentaires du tissu biologique, de façon à obtenir, pour chaque couche élémentaire, une grandeur d'intérêt différentielle ($\delta_{w,w'}(\Delta\zeta_{w,k})$);
- i) détermination d'une évolution, selon la profondeur du tissu biologique, des grandeurs d'intérêt différentielles ($\delta_{w,w'}(\Delta\zeta_{w,k})$).

2. Procédé selon l'une quelconque des revendications précédentes, dans lequel :

- les étapes a) à h) sont mises en oeuvre à différents instants de mesure ;
- l'étape i) comporte une formation d'un profil, pour chaque couche de élémentaire ($l_w$), chaque profil représentant une variation de la grandeur d'intérêt différentielle ($\delta_{w,w'}(\Delta\zeta_{w,k})$), dans ladite couche élémentaire, en fonction de l'instant de mesure (T) ;
- l'étape i) comporte un calcul d'un critère de corrélation entre différents profils, correspondant respectivement à différentes couches élémentaires, le critère de corrélation traduisant une corrélation entre les différents profils.

3. Procédé selon la revendication 2, comportant, suite à i) :

- j) détermination d'une couche élémentaire d'interface ($l_{w1/2}$), choisie parmi les différentes couches élémentaires, la couche élémentaire d'interface étant une couche de part et d'autre de laquelle le critère de corrélation franchit la valeur seuil, la couche élémentaire d'interface étant considérée comme formant une interface entre la couche superficielle ($20_1$) et la couche profonde ($20_2$), la couche élémentaire d'interface étant associée à un intervalle temporel d'interface ($d\tau_{1/2}$);
- k) à l'instant de référence et à un instant de mesure, à partir de chaque distribution temporelle résultant de c) établie à l'instant de mesure, calcul :

  • d'une première intensité temporelle ($I_{1,T}(\lambda_i)$), comportant une contribution, à la distribution temporelle, de photons détectés antérieurement à l'intervalle temporel d'interface, lesdits photons étant considérés comme s'étant propagés uniquement dans la couche superficielle ;
  • d'une deuxième intensité temporelle ($I_{2,T}(\lambda_i)$), comportant une contribution, à la distribution de photons détectés postérieurement à l'intervalle temporel d'interface, lesdits photons étant considérés comme s'étant propagés dans la couche superficielle et dans la couche profonde ;

- l) calcul d'un premier ratio $\left(\dfrac{I_{1,T}}{I_{1,ref}}(\lambda_i)\right)$, à partir des premières intensités temporelles respectivement déterminées à l'instant de référence et à l'instant de mesure et calcul d'un deuxième ratio $\left(\frac{I_{2,T}}{I_{2,ref}}(\lambda_i)\right)$, à partir des deuxièmes intensités temporelles respectivement déterminées à l'instant de référence et à l'instant de mesure ;

- m) estimation des variations des concentrations d'oxyhémoglobine et de désoxyhémoglobine ($\Delta c_{2,1}, \Delta c_{2,2}$) ou de grandeurs proportionnelles auxdites variations ($\overline{L}_2 \Delta c_{2,1}, \overline{L}_2 \Delta c_{2,2}$), dans la couche profonde ($20_2$), entre l'instant de mesure et l'instant de référence, à partir des ratios résultant de l) ;

**4.** Procédé selon la revendication 3, comportant :

- o) estimation des variations des concentrations d'oxyhémoglobine et de désoxyhémoglobine ($\Delta c_{1,1}, \Delta c_{1,1}$), ou de grandeurs proportionnelles auxdites variations ($\overline{L}_1 \Delta c_{1,1}, \overline{L}_1 \Delta c_{1,2}$), dans la couche superficielle ($20_1$) entre l'instant de mesure et l'instant de référence, à partir des ratios résultant de m).

**5.** Procédé selon l'une quelconque des revendications précédentes, dans lequel dans l'étape h), la couche de base est :

- une couche élémentaire prédéterminée ;
- ou une couche élémentaire de rang différent de la couche élémentaire considéré, l'écart entre le rang de la couche élémentaire de base et la couche élémentaire considéré étant constant.

**6.** Procédé selon l'une quelconque des revendications précédentes, dans lequel la source de lumière est une source laser.

**7.** Dispositif, destiné à détecter une survenue d'une occlusion veineuse ou artérielle dans un tissu biologique (20), entre au moins un instant de mesure (T) et un instant de référence $(T_{ref})$, le tissu biologique étant délimité par une surface (21), le dispositif comportant :

- une source de lumière impulsionnelle (10), la source de lumière étant disposée en regard de la surface du tissu biologique, la source de lumière étant configurée pour émettre un faisceau d'illumination (11), formant une zone d'illumination (12), à la surface du tissu biologique, la source de lumière étant configurée pour émettre simultanément ou successivement le faisceau d'illumination dans deux longueurs d'onde d'illumination différentes l'une de l'autre, chaque longueur d'onde s'étendant dans une bande spectrale d'absorption de l'oxyhémoglobine et/ou de la désoxyhémoglobine ;
- un photodétecteur (16), configuré pour détecter des photons rétrodiffusés par le tissu biologique, après s'être propagés dans le tissu biologique, les photons rétrodiffusés émanant d'une zone de détection (14) à la surface du tissu biologique ;
- une carte de comptage (17), destinée à établir une distribution temporelle ($I_T(\lambda_i), I_{Ref}(\lambda_i)$), représentant un nombre de photons détectés par le photodétecteur en fonction du temps, à chaque longueur d'onde ;
- une unité de traitement (18), programmée pour mettre en oeuvre les étapes d) à i) d'un procédé selon l'une quelconque des revendications précédentes, à partir des distributions temporelles établies par la carte de comptage à chaque longueur d'onde, à l'instant de mesure ou à chaque instant de mesure.

**Patentansprüche**

**1.** Verfahren zur Überwachung der Vaskularisierung eines biologischen Gewebes zwischen mindestens einem Messzeitpunkt (T) und einem Bezugszeitpunkt ($T_{ref}$), wobei das biologische Gewebe von einer Oberfläche (21) begrenzt wird, wobei das biologische Gewebe eine Oberflächenschicht ($20_1$) und eine tiefe Schicht ($20_2$) enthält, wobei die Oberflächenschicht sich zwischen der Oberfläche des Gewebes und der tiefen Schicht erstreckt, wobei das Verfahren aufweist:

- a) Beleuchtung des biologischen Gewebes durch eine Impulslichtquelle (10), wobei die Lichtquelle gegenüber der Oberfläche des biologischen Gewebes angeordnet ist, wobei die Lichtquelle einen Beleuchtungsstrahl (11) emittiert, der eine Beleuchtungszone (12) an der Oberfläche des biologischen Gewebes bildet, wobei der Beleuchtungsstrahl gleichzeitig oder nacheinander auf zwei voneinander unterschiedlichen Beleuchtungswellenlängen emittiert wird, wobei jede Wellenlänge sich in einem Absorptionsspektralband von Oxyhämoglobin und/oder von Desoxyhämoglobin erstreckt;
- b) bei jeder Wellenlänge, Erkennung von vom biologischen Gewebe rückgestreuten Photonen, nachdem sie sich im biologischen Gewebe ausgebreitet haben, durch einen Photodetektor (16), wobei die erkannten rückgestreuten Photonen von einer Erkennungszone (14) an der Oberfläche des biologischen Gewebes kommen ;
- c) bei jeder Wellenlänge, Erhalt einer zeitlichen Verteilung ($I_T(\lambda_i)$, $I_{Ref}(\lambda_i)$), die eine Anzahl von vom Photodetektor abhängig von der Zeit erkannten Photonen darstellt, wobei die zeitliche Verteilung sich gemäß einer Dauer ausgehend von einem Ursprungszeitpunkt erstreckt;
wobei die Schritte a) bis c) zum Bezugszeitpunkt und zum Messzeitpunkt oder zu jedem Messzeitpunkt ausgeführt werden ;
wobei das Verfahren **dadurch gekennzeichnet ist, dass** es zum Messzeitpunkt oder zu jedem Messzeitpunkt und zum Bezugszeitpunkt aufweist:

- d) Segmentierung der Dauer jeder zeitlichen Verteilung in Erkennungsintervalle ($d\tau_w$), wobei jedem Erkennungsintervall ein Rang (w) zugeordnet ist, wobei der Rang um so höher ist, je weiter das Erkennungsintervall vom Ursprungszeitpunkt der zeitlichen Verteilung entfernt ist, wobei jedes Erkennungsintervall einer Elementarschicht ($l_w$) zugeordnet ist, die sich in einer Tiefe ($d_w$) im biologischen Gewebe erstreckt;
- e) für jede Elementarschicht ($l_w$), ausgehend von jeder aus c) resultierenden zeitlichen Verteilung, die zum Messzeitpunkt und zum Bezugszeitpunkt erstellt werden, Bestimmung von zeitlichen Intensitäten ($I_{w,T}$, $I_{w,ref}$), entsprechend einer Anzahl von während jedes Erkennungsintervalls ($d\tau_w$) erkannten Photonen ;

und dass es zum Messzeitpunkt oder zu jedem Messzeitpunkt aufweist

- f) für jedes Erkennungsintervall ($d\tau_w$), Berechnung eines Verhältnisses zwischen der zeitlichen Intensität zum Messzeitpunkt und der zeitlichen Intensität zum Bezugszeitpunkt ;
- g) ausgehend von den aus f) resultierenden Verhältnissen, die für jede Wellenlänge erstellt werden, Berechnung einer interessierenden Größe ($\Delta\xi_{w,k}$) für jede jedem Erkennungsintervall zugeordnete Elementarschicht, wobei die interessierende Größe für eine Änderung der Konzentration von Hämoglobin in der Elementarschicht zwischen dem Messzeitpunkt und dem Bezugszeitpunkt repräsentativ ist ;
- h) für jede Elementarschicht ($l_w$), Vergleich der in g) berechneten interessierenden Größe mit einer interessierenden Basisgröße, berechnet für eine Basiselementarschicht, wobei die Basiselementarschicht eine der Elementarschichten des biologischen Gewebes ist, um für jede Elementarschicht eine differenzielle interessierende Größe ($\delta_{w,w'}(\Delta\xi_{w,k})$) zu erhalten;
- i) Bestimmung einer Entwicklung, gemäß der Tiefe des biologischen Gewebes, der differenziellen interessierenden Größen ($\delta_{w,w'}(\Delta\xi_{w,k})$).

2.  Verfahren nach einem der vorhergehenden Ansprüche, wobei:

    - die Schritte a) bis h) zu verschiedenen Messzeitpunkten durchgeführt werden ;
    - der Schritt i) eine Bildung eines Profils für jede Elementarschicht ($l_w$) aufweist, wobei jedes Profil eine Änderung der interessierenden differenziellen Größe ($\delta_{w,w'}(\Delta\xi_{w,k})$) in der Elementarschicht abhängig vom Messzeitpunkt (T) darstellt;
    - der Schritt i) eine Berechnung eines Korrelationskriteriums zwischen verschiedenen Profilen aufweist, die verschiedenen Elementarschichten entsprechen, wobei das Korrelationskriterium eine Korrelation zwischen den verschiedenen Profilen ausdrückt.

3.  Verfahren nach Anspruch 2, das nach i) aufweist:

    - j) Bestimmung einer Schnittstellen-Elementarschicht ($l_{w1/2}$), ausgewählt unter den verschiedenen Elementarschichten, wobei die Schnittstellen-Elementarschicht eine Schicht ist, auf deren beiden Seiten das Korrelationskriterium den Schwellwert überschreitet, wobei die Schnittstellen-Elementarschicht als eine Schnittstelle zwischen der Oberflächenschicht ($20_1$) und der tiefen Schicht ($20_2$) bildend angesehen wird, wobei die Schnittstellen-Elementarschicht einem zeitlichen Schnittstellenintervall ($d\tau_{1/2}$) zugeordnet ist ;
    - k) zum Bezugszeitpunkt und zu einem Messzeitpunkt, ausgehend von jeder aus c) resultierenden zeitlichen Verteilung c), die zum Messzeitpunkt erstellt wird, Berechnung :

      • einer ersten zeitlichen Intensität ($I_{1,T}(\lambda_i)$), die einen Beitrag zur zeitlichen Verteilung von vor dem zeitlichen Schnittstellenintervall erkannten Photonen aufweist, wobei von den Photonen angenommen wird, dass sie sich nur in der Oberflächenschicht ausgebreitet haben ;

• einer zweiten zeitlichen Intensität ($I_{2,T}(\lambda_i)$), die einen Beitrag zur Verteilung von nach dem zeitlichen Schnittstellenintervall erkannten Photonen aufweist, wobei von den Photonen angenommen wird, dass sie sich in der Oberflächenschicht und in der tiefen Schicht ausgebreitet haben ;

- l) Berechnung eines ersten Verhältnisses $\left(\frac{I_{1,T}}{I_{1,ref}}(\lambda_i)\right)$ ausgehend von den ersten im Bezugszeitpunkt bzw. Messzeitpunkt bestimmten zeitlichen Intensitäten und Berechnung eines zweiten Verhältnisses $\left(\frac{I_{2,T}}{I_{2,ref}}(\lambda_i)\right)$ ausgehend von den zweiten im Bezugszeitpunkt bzw. Messzeitpunkt bestimmten zeitlichen Intensitäten ;

- m) Schätzung der Änderungen der Konzentrationen von Oxyhämoglobin und von Desoxyhämoglobin ($\Delta c_{2,1}$, $\Delta c_{2,2}$) oder von Größen proportional zu den Änderungen ($\overline{L}_2 \Delta c_{2,1}$, $\overline{L}_2 \Delta c_{2,2}$) in der tiefen Schicht ($20_2$) zwischen dem Messzeitpunkt und dem Bezugszeitpunkt ausgehend von den aus l) resultierenden Verhältnissen.

4. Verfahren nach Anspruch 3, das aufweist:

- o) Schätzung der Änderungen der Konzentrationen von Oxyhämoglobin und von Desoxyhämoglobin ($\Delta c_{1,1}$, $\Delta c_{1,1}$) oder von Größen proportional zu den Änderungen ($\overline{L}_1 \Delta c_{1,1}$, $\overline{L}_1 \Delta c_{1,2}$) in der Oberflächenschicht ($20_1$) zwischen dem Messzeitpunkt und dem Bezugszeitpunkt ausgehend von den aus m) resultierenden Verhältnissen.

5. Verfahren nach einem der vorhergehenden Ansprüche, wobei im Schritt h) die Basisschicht ist :

- eine vorbestimmte Elementarschicht ;
- oder eine Elementarschicht eines anderen Rangs als die betrachtete Elementarschicht, wobei die Abweichung zwischen dem Rang der Basiselementarschicht und der betrachteten Elementarschicht konstant ist.

6. Verfahren nach einem der vorhergehenden Ansprüche, wobei die Lichtquelle eine Laserquelle ist.

7. Vorrichtung, dazu bestimmt, ein Auftreten eines Venen- oder Arterienverschlusses in einem biologischen Gewebe (20) zwischen mindestens einem Messzeitpunkt (T) und einem Bezugszeitpunkt ($T_{ref}$) zu erkennen, wobei das biologische Gewebe von einer Oberfläche (21) begrenzt wird, wobei die Vorrichtung aufweist:

- eine Impulslichtquelle (10), wobei die Lichtquelle gegenüber der Oberfläche des biologischen Gewebes angeordnet ist, wobei die Lichtquelle konfiguriert ist, einen Beleuchtungsstrahl (11) zu emittieren, der eine Beleuchtungszone (12) an der Oberfläche des biologischen Gewebes formt, wobei die Lichtquelle konfiguriert ist, gleichzeitig oder nacheinander den Beleuchtungsstrahl auf zwei voneinander unterschiedlichen Beleuchtungswellenlängen zu emittieren, wobei jede Wellenlänge sich in einem Absorptionsspektralband des Oxyhämoglobins und/oder des Desoxyhämoglobins erstreckt;
- einen Photodetektor (16), der konfiguriert ist, vom biologischen Gewebe rückgestreute Photonen zu erkennen, nachdem sie sich im biologischen Gewebe ausgebreitet haben, wobei die rückgestreuten Photonen von einer Erkennungszone (14) an der Oberfläche des biologischen Gewebes kommen ;
- eine Zählkarte (17), dazu bestimmt, eine zeitliche Verteilung ($I_T(\lambda_i)$, $I_{Ref}(\lambda_i)$) zu erstellen, die eine Anzahl von vom Photodetektor auf jeder Wellenlänge abhängig von der Zeit erkannten Photonen darstellt;
- eine Verarbeitungseinheit (18), die programmiert ist, die Schritte d) bis i) eines Verfahrens nach einem der vorhergehenden Ansprüche ausgehend von den von der Zählkarte auf jeder Wellenlänge erstellten zeitlichen Verteilungen zum Messzeitpunkt oder zu jedem Messzeitpunkt durchzuführen.

**Claims**

1. Method for monitoring the vascularization of a biological tissue between at least a measurement time (T) and a reference time ($T_{ref}$), the biological tissue being bounded by a surface (21), the biological tissue comprising a surface

layer ($20_1$) and a deep layer ($20_2$), the surface layer lying between the surface of the tissue and the deep layer, the method comprising:

- a) illuminating the biological tissue with a pulsed light source (10), the light source being placed facing the surface of the biological tissue, the light source emitting an illumination beam (11) forming an illumination zone (12) on the surface of the biological tissue, the illumination beam being emitted simultaneously or successively at two illumination wavelengths that are different from each other, each wavelength lying in an absorption spectral band of oxyhaemoglobin and/or deoxyhaemoglobin;
- b) at each wavelength, detecting photons backscattered by the biological tissue, after having propagated through the biological tissue, by means of a photodetector (16), the detected backscattered photons emanating from a detection zone (14) on the surface of the biological tissue;
- c) at each wavelength, obtaining a time-dependent distribution ($I_T(\lambda_i)$, $I_{Ref}(\lambda_i)$), representing a number of photons detected by the photodetector as a function of time, the time-dependent distribution spanning a duration, from an initial time;

steps a) to c) being performed at the reference time and at the measurement time or each measurement time; the method being **characterized in that** it comprises, at the measurement time or each measurement time and at the reference time:

- d) segmenting the duration of each time-dependent distribution into detection intervals ($d\tau_w$), each detection interval being associated with a rank (w), the rank increasing with the separation of the detection interval from the initial time of the time-dependent distribution, each detection interval being associated with an elementary layer ($l_w$) lying at a depth ($d_w$) in the biological tissue;
- e) for each elementary layer ($l_w$), on the basis of each time-dependent distribution resulting from c), established at the measurement time and at the reference time, determining time-dependent intensities ($I_{w,T}$, $I_{w,ref}$) corresponding to a number of photons detected during each detection interval ($d\tau_w$);

and **in that** it comprises, at the measurement time or at each measurement time:

- f) for each detection interval ($d\tau_w$), computing a ratio between the time-dependent intensity at the measurement time and the time-dependent intensity at the reference time;
- g) on the basis of the ratios resulting from f), established for each wavelength, computing a quantity of interest ($\Delta\zeta_{w,k}$) for each elementary layer associated with each detection interval, respectively, the quantity of interest being representative of a variation in the haemoglobin concentration, in said elementary layer, between the measurement time and the reference time;
- h) for each elementary layer ($l_w$), comparing the quantity of interest, computed in g), with a base-line quantity of interest, computed for a base-line elementary layer, the base-line elementary layer being one of the elementary layers of the biological tissue, so as to obtain, for each elementary layer, a differential quantity of interest ($\delta_{w,w'}(\Delta\zeta_{w,k})$);
- i) determining a variation, as a function of depth of the biological tissue, of the differential quantities of interest ($\delta_{w,w'}(\Delta\zeta_{w,k})$).

2. Method according to any one of the preceding claims, wherein:

- steps a) to h) are implemented at various measurement times;
- step i) comprises forming a profile, for each elementary layer ($l_w$), each profile representing a variation in the differential quantity of interest ($\delta_{w,w'}(\Delta\zeta_{w,k})$), in said elementary layer, as a function of the measurement time ($T$);
- step i) comprises computing a correlation criterion quantifying the correlation between various profiles, corresponding to various elementary layers, respectively, the correlation criterion expressing a correlation between the various profiles.

3. Method according to Claim 2, comprising, following i):

- j) determining an interface elementary layer ($l_{w1/2}$) chosen from the various elementary layers, the interface elementary layer being a layer on either side of which the correlation criterion is beyond the threshold value, the interface elementary layer being considered to form an interface between the surface layer ($20_1$) and the deep layer ($20_2$), the interface elementary layer being associated with an interface time interval ($d\tau_{1/2}$);
- k) at the reference time and at a measurement time, on the basis of each time-dependent distribution resulting from c) established at the measurement time, computing:

• a first time-dependent intensity ($I_{1,T}(\lambda_i)$) comprising a contribution, to the time-dependent distribution, of photons detected prior to the interface time interval, said photons being considered to have propagated only through the surface layer;

• a second time-dependent intensity ($I_{2,T}(\lambda_i)$) comprising a contribution, to the distribution, of photons detected subsequently to the interface time interval, said photons being considered to have propagated through the surface layer and through the deep layer;

- l) computing a first ratio $\left(\dfrac{I_{1,T}}{I_{1,ref}}(\lambda_i)\right)$, on the basis of the first time-dependent intensities determined at the reference time and at the measurement time, respectively, and computing a second ratio $\left(\dfrac{I_{2,T}}{I_{2,ref}}(\lambda_i)\right)$, on the basis of the second time-dependent intensities determined at the reference time and at the measurement time, respectively;

- m) estimating the variations in the oxyhaemoglobin and deoxyhaemoglobin concentrations ($\Delta c_{2,1}$, $\Delta c_{2,2}$) or quantities ($\overline{L}_2\Delta c_{2,1}$, $\overline{L}_2\Delta c_{2,2}$) proportional to said variations, in the deep layer ($20_2$), between the measurement time and the reference time, on the basis of the ratios resulting from l).

4. Method according to Claim 3, comprising:

- o) estimating the variations in the oxyhaemoglobin and deoxyhaemoglobin concentrations ($\Delta c_{1,1}$, $\Delta c_{1,1}$), or quantities ($\overline{L}_1\Delta c_{1,1}$, $\overline{L}_1\Delta c_{1,2}$) proportional to said variations, in the surface layer ($20_1$) between the measurement time and the reference time, on the basis of the ratios resulting from m).

5. Method according to any one of the preceding claims, wherein, in step h), the base layer is:

- a predetermined elementary layer;
- or an elementary layer of different rank from the elementary layer in question, the difference between the rank of the base elementary layer and the elementary layer in question being constant.

6. Method according to any one of the preceding claims, wherein the light source is a laser source.

7. Device intended to detect occurrence of a veinous or arterial occlusion in a biological tissue (20), between at least one measurement time ($T$) and one reference time ($T_{ref}$), the biological tissue being bounded by a surface (21), the device comprising:

- a pulsed light source (10), the light source being placed facing the surface of the biological tissue, the light source being configured to emit an illumination beam (11), forming an illumination zone (12) on the surface of the biological tissue, the light source being configured to emit the illumination beam simultaneously or successively at two illumination wavelengths that are different from each other, each wavelength lying in an absorption spectral band of oxyhaemoglobin and/or deoxyhaemoglobin;
- a photodetector (16), configured to detect photons backscattered by the biological tissue, after having propagated through the biological tissue, the backscattered photons emanating from a detection zone (14) on the surface of the biological tissue;
- a counting board (17), intended to establish a time-dependent distribution ($I_T(\lambda_i)$, $I_{Ref}(\lambda_i)$) representing a number of photons detected by the photodetector as a function of time, at each wavelength;
- a processing unit (18), programmed to implement steps d) to i) of a method according to any one of the preceding claims, on the basis of the time-dependent distributions established by the counting board at each wavelength, at the measurement time or at each measurement time.

**Fig. 1A**

**Fig. 1B**

**Fig. 1C**

**Fig. 1D**

**Fig. 2A**

**Fig. 2B**

$$100$$

$$I_T(\lambda_i), I_{ref}(\lambda_i)$$

$$d\tau_w \longrightarrow 110$$

$$I_{w,T}(\lambda_i), I_{w,ref}(\lambda_i)$$

$$120$$

$$\Delta c_{w,k}$$

$$130$$

$$\delta_{w,w'}(\Delta c_{w,k})$$

$$I_T(\lambda_i), I_{Ref}(\lambda_i) \longrightarrow 140$$

$$\tau_1 \quad \tau_2$$

$$150$$

$$\Delta c_{k,1} \quad \Delta c_{k,2}$$

**Fig. 3**

**Fig. 4A**

**Fig. 4B**

**Fig. 5A**

**Fig. 5B**

Fig. 5C

Fig. 5D

Fig. 5E

**Fig. 6A**

**Fig. 6B**

**Fig. 6C**

**Fig. 7A**

**Fig. 7B**

**Fig. 8A**

**Fig. 8B**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- EP 2762064 A
- EP 3054282 A
- EP 3054281 A
- EP 3311138 A
- EP 2605002 A

**Littérature non-brevet citée dans la description**

- **SELB. J et al.** Improved sensitivity to cerebral hemodynamics during brain activation with a time-gated optical system: Analytical model and experimental validation. *Journal of Biomerical Optics,* 2007, vol. 10 (1
- **CONTINI, D et al.** Novel method for depth-resolved brain functional imaging by time-domain NIRS. *Proc. SPIE 6629, Diffuse Optical imaging of Tissue,* 12 Juillet 2007, 662908
- **ZUCCHELLI L ; CONTINI D, RE R ; TORRICELLI A ; SPINELLI L.** Method for the discrimination of superficial and deep absorption variations by time domain fNIRS. *Biomed Opt Express,* 20 Novembre 2013, vol. 4 (12), 2893-2910
- **L. HERVÉ et al.** Time-domaine diffuse optical tomography processing by suing the Mellin-Laplace transform. *Applied Optics,* 2012, vol. 51 (25), 5978-5988
- **J. ZAOUAOUI.** Chromophore décomposition in multispectral time-resolved diffuse optical tomography. *Biomédical optics Express,* 2017, vol. 8 (10), 4772